# EUROPÄISCHE PATENTSCHRIFT

(11) **EP 4 141 846 B1**
(45) Veröffentlichungstag und Bekanntmachung des Hinweises auf die Patenterteilung: **03.12.2025**
(21) Anmeldenummer: 22190920.3
(22) Anmeldetag: 18.08.2022
(51) Int. Cl.: G09B 23/28, G09B 23/32, A61M 16/00

(54) **SYSTEM ZUR SIMULATION DER ATMUNG EINES LEBEWESENS**
SYSTEM FOR SIMULATING THE BREATHING OF A LIVING BEING
SYSTÈME DE SIMULATION DE LA RESPIRATION D'UN ORGANISME VIVANT

(30) Priorität: 26.08.2021 DE 102021004375
(43) Veröffentlichungstag der Anmeldung: 01.03.2023
(73) Patentinhaber: Löwenstein Medical Technology S.A., 2557 Luxembourg (LU)
(72) Erfinder: Borm, Petrus Johannes Josephus, 5627 HP Eindhoven (NL)
(74) Vertreter: Löwenstein Medical IP

(56) Entgegenhaltungen:
- FR-A1- 2 704 762
- US-A- 5 890 908
- US-A1- 2004 110 117

## Beschreibung

Die Erfindung betrifft eine Vorrichtung und ein Verfahren zur Simulation der Atmung eines Lebewesens.

Neue Funktionen von Beatmungsgeräten und auch anderen medizinischen Geräten basieren häufig zunächst auf theoretischen Überlegungen zu bestimmten Situationen. Insbesondere die Erkennung von bestimmten Situationen durch beispielsweise ein Beatmungsgerät stützt sich meist auf solche Überlegungen. Um solche Funktionen zu testen, gibt es einen Bedarf an Simulatoren, welche eine Atmung simulieren.

Auch kann es immer wieder zu Qualitätschecks von Geräten kommen, beispielsweise auch von Diagnosegeräten, ob diese eine Situation richtig erkennen. Auch hier kommen Simulatoren zum Einsatz. Letztlich sind Simulatoren auch in der Ausbildung von Bedeutung. Beispielsweise kann es möglich sein, insbesondere eher seltene Vorkommnisse darzustellen und zu üben.

Die aus dem Stand der Technik bekannten Simulatoren bieten häufig eine rudimentäre Abbildung der Atmung eines Lebewesens, sodass nur grundlegende Atmungssituationen dargestellt werden können. Auch verlassen sich diese bekannten Simulatoren hauptsächlich auf eine mechatronische Gestaltung der Simulation, ohne eine umfassendere Grundlage hinter die Abläufe zu legen.

US 2004/0110117 Al beschreibt eine simulierte Lunge zur Verwendung bei simulierten medizinischen Verfahren in Echtzeit. Die Lunge umfasst eine Einrichtung zum kontinuierlichen Auswerten eines von einem Drucksensor erzeugten Drucksignals, um einen eintretenden und einen austretenden Gasfluss mit einer gewünschten Atemflussrate zu synchronisieren. Das gewünschte Druckniveau hängt dabei ab von einem zeit- und ereignisbasierten Skript, einem Computermodell oder einer Kombination aus beidem, basierend auf einem physiologischen Zustand eines simulierten Patienten.

US 5 890 908 A beschreibt eine Vorrichtung und ein Verfahren zum Injizieren und Verflüchtigen eines flüchtigen Medikaments in einem Patientensimulator, einschließlich einer Puppe, einer Gasversorgung und einer Vorrichtung zum Verflüchtigen des Medikaments. Dabei wird eine simulierte Reaktion auf das Arzneimittel berechnet und eine mit der Puppe verbundene Ausgabevorrichtung betätigt, um die Wirkung des Arzneimittels zu simulieren.

Die Aufgabe der vorliegenden Erfindung ist daher, ein System bereitzustellen, welches eine einfache Simulation der Atmung eines Lebewesens ermöglicht. Die Aufgabe wird durch ein System und ein Verfahren gemäß den unabhängigen Ansprüchen gelöst.

Die Erfindung betrifft ein System zur Simulation der Atmung eines Lebewesens umfassend zumindest ein Gasmodul und ein Steuermodul, wobei das Steuermodul dazu eingerichtet und ausgebildet ist, in einem ersten Simulationsteil die Atmung eines Lebewesens mathematisch zu simulieren und in einem zweiten Simulationsteil anhand der mathematischen Simulation aus dem ersten Simulationsteil das Gasmodul zu steuern.

In manchen Ausführungsformen kennzeichnet das System, dass das Steuermodul eine Simulationseinheit umfasst, welche dazu eingerichtet und ausgebildet ist, die Atmung mathematisch zu simulieren.

In manchen Ausführungsformen kennzeichnet das System, dass das Steuermodul dazu eingerichtet und ausgebildet ist, das Gasmodul so anzusteuern, dass in dem zweiten Simulationsteil die mathematische Simulation des ersten Simulationsteils in eine physikalische Simulation der Atmung eines Lebewesens umgesetzt wird.

In manchen Ausführungsformen kennzeichnet das System, dass das Gasmodul zumindest eine Exspirationseinheit und zumindest eine Inspirationseinheit umfasst, wobei die Exspirationseinheit dazu eingerichtet ist eine Exspiration eines Lebewesens zu simulieren und die Inspirationseinheit dazu eingerichtet ist eine Inspiration eines Lebewesens zu simulieren.

In manchen Ausführungsformen kennzeichnet das System, dass die Simulationseinheit dazu ausgebildet ist, den Druck zu berechnen und/oder zu simulieren, welcher durch das simulierte Lebewesen in der Lunge erzeugt wird.

In manchen Ausführungsformen kennzeichnet das System, dass das Gasmodul dazu ausgebildet und eingerichtet ist, den Druck, welcher durch das simulierte Lebewesen in der Lunge erzeugt wird, physikalisch zu simulieren.

In manchen Ausführungsformen kennzeichnet das System, dass das Gasmodul über einen Anschluss mit einem Beatmungsgerät verbindbar ist.

In manchen Ausführungsformen kennzeichnet das System, dass die Exspirationseinheit zumindest eine Gasquelle und/oder zumindest ein Gebläse umfasst.

In manchen Ausführungsformen kennzeichnet das System, dass die Inspirationseinheit dazu eingerichtet und ausgebildet ist einen Unterdruck zu erzeugen.

In manchen Ausführungsformen kennzeichnet das System, dass die Exspirationseinheit eine Mehrzahl von Gasquellen umfasst, wobei die Exspirationseinheit dazu eingerichtet und ausgebildet ist ein Gasgemisch bereitzustellen.

In manchen Ausführungsformen kennzeichnet das System, dass die Exspirationseinheit dazu eingerichtet und ausgebildet ist, auf Basis der mathematischen Simulation eine Gasmischung bereitzustellen, welche einer Gaszusammensetzung der ausgeatmeten Luft eines Lebewesens entspricht.

In manchen Ausführungsformen kennzeichnet das System, dass in dem Gasmodul ein Gebläse angeordnet ist, wobei über eine Schaltung von im Gasmodul angeordneten Ventilen und Bypass-Leitungen, das Gebläse sowohl als Exspirationseinheit als auch als Inspirationseinheit dient.

In manchen Ausführungsformen kennzeichnet das System, dass das im Gasmodul zumindest ein pneumatischer Widerstand angeordnet ist.

In manchen Ausführungsformen kennzeichnet das System, dass das System eine Sensorik umfasst, welche dazu eingerichtet und ausgebildet ist, Werte der Atmung zu erfassen.

In manchen Ausführungsformen kennzeichnet das System, dass das Steuermodul dazu eingerichtet und ausgebildet ist, die über die Sensorik aufgenommenen Werte mit in die mathematische Simulation einzubeziehen. In manchen Ausführungsformen kennzeichnet das System, dass das Steuermodul eine Auswerteeinheit umfasst, welche dazu eingerichtet und ausgebildet ist, die über die Sensorik erfassten Werte auszuwerten und/oder zu analysieren.

In manchen Ausführungsformen kennzeichnet das System, dass die Auswerteeinheit dazu eingerichtet und ausgebildet ist, die über die Sensorik erfassten Werte dahingehend zu analysieren, ob die mathematische Simulation durch das Gasmodul korrekt umgesetzt wird.

In manchen Ausführungsformen kennzeichnet das System, dass die Inspirationseinheit und die Exspirationseinheit als eine kombinierte Einheit ausgebildet sind.

In manchen Ausführungsformen kennzeichnet das System, dass das System über eine Eingabeeinheit verfügt, über welche Daten, Werte und/oder Informationen eingegeben werden, wobei die Daten, Werte und/oder Informationen zumindest teilweise als Vorgaben zur mathematischen Simulation dienen.

In manchen Ausführungsformen kennzeichnet das System, dass die Eingabeeinheit dazu eingerichtet und ausgebildet ist, Werte und/oder Daten und/oder Informationen aus der Auswerteeinheit in die Simulationseinheit einzugeben.

In manchen Ausführungsformen kennzeichnet das System, dass die Eingabeeinheit mit zumindest einem Eingabemodul verbunden ist, wobei über das Eingabemodul die aktuelle Simulation angezeigt wird.

In manchen Ausführungsformen kennzeichnet das System, dass das System einen Atemgasanfeuchter und/oder eine Atemgasheizung umfasst.

In manchen Ausführungsformen kennzeichnet das System, dass das Steuermodul dazu eingerichtet und ausgebildet ist, ein Beatmungsgerät anhand der mathematischen Simulation zumindest teilweise zu steuern, wobei das Beatmungsgerät mit einer realen Person verbunden ist.

In manchen Ausführungsformen kennzeichnet das System, dass das System mit Patientensimulatoren kombinierbar ist.

In manchen Ausführungsformen kennzeichnet das System, dass die Simulation der Atmung auch die Simulation weiterer physiologischer Parameter umfasst.

In manchen Ausführungsformen kennzeichnet das System, dass ein mathematisch simulierter Atemfluss durch zumindest ein Gebläse physikalisch simuliert wird und die simulierte Gaszusammensetzung durch zumindest eine Gasquelle erreicht wird.

Die Erfindung betrifft auch ein Verfahren zur Simulation der Atmung eines Lebewesens, wobei in einem Verfahrensschritt die Atmung des Lebewesens in einem ersten Simulationsteil durch eine mathematische Simulation simuliert wird und in einem weiteren Verfahrensschritt in einem zweiten Simulationsteil ein Gasmodul auf Basis der mathematischen Simulation angesteuert wird.

In manchen Ausführungsformen kennzeichnet das Verfahren, dass die mathematische Simulation unmittelbar in Steuerbefehle umgesetzt wird und das Gasmodul anhand der Steuerbefehle gesteuert wird.

In manchen Ausführungsformen kennzeichnet das Verfahren, dass in einem Verfahrensschritt über Sensoren Messwerte zur Atmung aufgenommen werden und mit in die mathematische Simulation einbezogen werden.

In manchen Ausführungsformen kennzeichnet das Verfahren, dass die mathematische Simulation automatisch anhand der aufgenommenen Messwerte angepasst und/oder abgeändert wird.

In manchen Ausführungsformen kennzeichnet das Verfahren, dass die Messwerte zur Atmung einer realen Person für die mathematische Simulation verwendet werden.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Im Zuge der Erfindung sind unter Lebewesen insbesondere solche Lebewesen zu verstehen, welche Gas, zum Beispiel Luft, atmen. Insbesondere sind unter diesen Lebewesen Säugetiere, insbesondere Menschen, zu verstehen. In manchen Ausführungsformen bezieht sich die Erfindung explizit auf die Simulation der Atmung von Menschen.

Unter einer Beatmung ist eine Unterstützung und/oder Vorgabe der Atmung durch eine externe Quelle zu verstehen. Die externe Quelle kann dabei beispielsweise eine maschinelle Beatmung, etwa über ein Beatmungsgerät, und/oder eine manuelle Beatmung, etwa über eine Mund-zu-Mund-Beatmung oder einen Beatmungsbeutel, und/oder die Versorgung mit Gas über eine Druckluftflasche sein.

Unter einem Beatmungsgerät ist jedwedes Gerät zu verstehen, welches einen Anwender oder Patienten bei der natürlichen Atmung unterstützt, die Beatmung des Anwenders bzw. Lebewesens (z.B. Patient und/oder Neugeborener und/oder Frühgeborene) übernimmt und/oder zur Atemtherapie dient und/oder anderweitig die Atmung des Anwenders bzw. Patienten beeinflusst. Darunter fallen zum Beispiel, aber nicht ausschließend, CPAP- sowie BiLevel-Geräte, Narkose- bzw. Anästhesiegeräte, Atemtherapiegeräte, (klinische, außerklinische oder Notfall-) Beatmungsgeräte, Highflow-Therapiegeräte und Hustenmaschinen. Beatmungsgeräte können auch als Diagnosegeräte für die Beatmung verstanden werden. Diagnosegeräte können dabei allgemein zur Erfassung von medizinischen und/oder atmungsbezogenen Parametern eines Lebewesens dienen. Darunter fallen auch Geräte, welche medizinische Parameter von Patienten in Kombination mit der Atmung oder ausschließlich die Atmung betreffend, erfassen und optional verarbeiten können.

Als Patienteninterface kann, soweit nicht ausdrücklich anders beschrieben, jegliches Peripheriegerät verstanden werden, welches zur Interaktion, insbesondere zu Therapie- oder Diagnosezwecken, der Messeinrichtung mit einem Lebewesen gedacht ist. Insbesondere kann ein Patienteninterface als Maske eines Beatmungsgerätes bzw. eine mit dem Beatmungsgerät verbundene Maske verstanden werden. Diese Maske kann eine Full-Face Maske, also Nase und Mund umschließende, oder eine Nasenmaske, also eine nur die Nase umschließende Maske, sein. Auch Trachealtuben bzw. -kanülen und sogenannte Nasenbrillen können als Maske beziehungsweise Patienteninterface eingesetzt werden. In manchen Fällen kann das Patienteninterface auch ein einfaches Mundstück, beispielsweise ein Rohr, sein, durch welches das Lebewesen/der Patient/der Anwender zumindest ausatmet und/oder einatmet.

Die Simulation der Atmung des Lebewesens durch das System teilt sich in zwei Simulationsteile auf. In einem ersten Simulationsteil wird die Atmung des Patienten mathematisch simuliert und in einem zweiten Simulationsteil wird auf Basis der mathematischen Simulation ein Gasmodul gesteuert.

Im zweiten Simulationsteil kann vorgesehen sein, dass das Gasmodul die mathematische Simulation in eine physikalische Simulation umsetzt.

Es kann ergänzend oder alternativ auch vorgesehen sein, dass im zweiten Simulationsteil die mathematische Simulation genutzt wird, um ein Beatmungsgerät zu steuern.

Die mathematische Simulation umfasst dabei zumindest eine computergestützte Berechnung der Lunge und/oder der Luftröhre bzw. die Atmung des simulierten Lebewesens, wobei zumindest die Druck- und/oder Flusssituation in der Lunge und/oder der Luftröhre und/oder der Atmung des Lebewesens berechnet bzw. simuliert wird. Die mathematische Simulation kann dabei auf Vorgaben und/oder Sensorwerten beruhen. Beispielsweise ist vorgesehen, dass die Atmung des Lebewesens durch Vorgaben zumindest hinsichtlich des Drucks und des Flusses des Gases simuliert werden. Auch kann alternativ oder ergänzend vorgesehen sein, dass Gewicht, Größe, Geschlecht, Körperfettanteil, Muskelanteil, Alter, Erkrankungen, Lungenvolumen, Zustand der Alveolen (kollabiert, überdehnt, geöffnet, zu welchen Anteilen), Sauerstoff/CO2 Austausch, Atemzugsvolumen, Atemfrequenz mit in die Simulation einbezogen werden. Weitere Vorgaben und/oder Sensorwerte und/oder Parameter, welche mit in die Simulation, insbesondere in die mathematische Simulation, einbezogen werden können sind beispielsweise Tidalvolumen, Aufnahme von Anästhesiegasen, Hustenereignissen und/oder -anstrengungen, Muskelanstrengungen im Zusammenhang mit der Lunge/Atmung, Compliance, Compliance-Änderung als Funktion der Zeit und/oder des Drucks, Aktivität/Bewegungen von Muskelgruppen, Fettgruppen, Herzfrequenz, Änderungen der Herzfrequenz, Blutverdünnung, Sauerstoff- und/oder CO2-Aufnahme/Abgabe im Körper, Körpertemperatur, Hyper- und Hypoventilation, Ausscheidung von Anästhesiegasen über Leber oder Niere.

Es kann auch vorgesehen sein, dass Vorgaben zum Verlauf der mathematischen Simulation eingegeben werden können. Beispielsweise können Atemprobleme bzw. Atemsituationen (Atemereignisse) wie Apnoen, Kollaps der Alveolen, Schnappatmung, erhöhte/verringerte Atemfrequenz, Situationen die einem Schnarchen gleichen, etc. vorprogrammiert werden. Diese können dann nach beispielsweise vorgegebenen Zeiten mathematisch simuliert werden. Auch kann ein Zufallsgenerator vorgesehen sein, welcher Atmungssituationen zufällig in die mathematische Simulation miteinbezieht. Beispielsweise kann vorgegeben sein, welche Atmungssituation(en) simuliert werden sollen, optional mit weiteren Einstellungen wie Ausprägung der Situation, und über den Zufallsgenerator werden die Situationen zufällig in die Simulation miteinbezogen. Auch kann vorgesehen sein, die Ausprägung über einen Zufallsgenerator vorgeben zu lassen.

Alternativ oder ergänzend reagiert die mathematische Simulation auf Sensorwerte. Beispielsweise wird durch ein Beatmungsgerät ein kontinuierlicher Druck (CPAP) ausgeübt, worauf die mathematische Simulation diesen Druck mit in die Berechnung einbezieht. Es kann beispielsweise vorgesehen sein, dass die mathematische Simulation nur auf Sensorwerte, insbesondere dem von einem Beatmungsgerät bereitgestellten Druck, reagiert.

Bei der auf der mathematischen Simulation basierenden Steuerung des Gasmoduls ist beispielsweise vorgesehen, dass das System mit einem Fluss auf den Druck des Beatmungsgerätes reagiert.

In einem Simulationsablauf kann auch vorgesehen sein, dass zunächst eine mathematische Simulation auf Basis von eingegebenen Vorgaben erfolgt und im weiteren Verlauf durch Sensorwerte angepasst wird. Auch kann vorgesehen sein, dass die mathematische Simulation sowohl die eingegebenen Vorgaben als auch Sensorwerte miteinbezieht. Beispielsweise kann vorgesehen sein, dass die mathematische Simulation auf die Sensorwerte reagiert, beispielsweise als Reaktion auf eine veränderte Arbeitsweise eines verbundenen Beatmungsgeräts.

Die Sensorwerte können alternativ oder ergänzend auch die Atmung einer realen Person bzw. eines realen Lebewesens betreffen. Beispielsweise kann anhand der gemessenen Sensorwerte der Atmung der realen Person die reale Person über die mathematische Simulation nachgestellt werden und optional auch ergänzende Werte simuliert werden.

Beispielsweise können Sensorwerte zu Druck, Fluss, Frequenz, Volumen und/oder Gaszusammensetzung aufgenommen werden, wobei anhand dieser Werte eine mathematische Simulation der Situation in der Lunge ermöglicht wird. Dabei kann die mathematische Simulation beispielsweise zusätzliche Werte, Daten und/oder Informationen, wie die Gaszusammensetzung in der Lunge und/oder einen Anteil an kollabierten/überdehnten/offenen Alveolen und/oder das Lungenvolumen und/oder weitere Parameter der Lunge berechnen.

In manchen Ausführungsformen kann vorgesehen sein, dass neben der Atmung auch weitere Körperfunktionen des Lebewesens, beispielsweise Körpertemperatur, Blutwerte und/oder Herzschlag, zumindest mathematisch simuliert werden. Gegebenenfalls kann eine solche mathematische Simulation direkt als Signale an angeschlossene Messgeräte weitergegeben werden und/oder es kann vorgesehen weitere physikalische Simulationsmodule anzuschließen, welche die jeweilige mathematische Simulation in eine physikalische Simulation umsetzen.

Es ist vorgesehen, dass verschiedene Aspekte der Atmung des Lebewesens durch die mathematische Simulation simuliert werden können. Insbesondere sind Druck und Fluss der Atmung simulierbar. In manchen Ausführungsformen ist zudem vorgesehen, dass auch Gaszusammensetzung, Atemfrequenz, Atemsituationen, Atemprobleme, Gasaustausch, etc. simuliert werden können.

Für das System ist weiter vorgesehen, dass im zweiten Simulationsteil ein Gasmodul anhand der mathematischen Simulation gesteuert wird. Das Gasmodul kann zum einen eine Vorrichtung sein, welche eine physikalische Simulation der Atmung bewirkt, zumindest einen Gasdruck und Gasfluss umfassend.

Auch Gaszusammensetzung, Atemfrequenz, Atemsituationen, Atemprobleme und/oder Gasaustausch und/oder weitere Aspekte der Atmung können über das Gasmodul physikalisch simulierbar sein.

Alternativ oder ergänzend kann das Gasmodul als Beatmungsgerät ausgebildet sein, sodass die mathematische Simulation dazu genutzt wird, die Steuerung des Beatmungsgerätes zumindest zu beeinflussen.

Es kann vorgesehen sein, dass das Beatmungsgerät auf Basis der mathematischen Simulation gesteuert wird.

Beispielsweise werden zumindest die zusätzlichen, über die mathematische Simulation berechneten, Werte/Daten/Informationen an das Beatmungsgerät weitergeleitet, sodass auf dieser Basis die Beatmung gegebenenfalls angepasst wird.

Werden neben der Atmung auch weitere Körperfunktionen mathematisch simuliert, kann vorgesehen sein, dass neben dem Gasmodul weitere physikalische Simulatoren im System angeordnet sind, welche die mathematische Simulation in eine physikalische Simulation umsetzen können.

Alternativ oder ergänzend kann vorgesehen sein, dass die mathematische Simulation zumindest in elektrische Signale umgewandelt werden, welche wiederum beispielsweise über Sensoren detektiert und/oder interpretiert werden können.

Ein Aspekt der Erfindung betrifft ein System, welches dazu eingerichtet und ausgebildet ist, die Atmung eines Lebewesens zu simulieren. Die Simulation der Atmung betrifft dabei die zumindest teilweise selbstständige Atmung und/oder die zumindest teilweise vorgegebene Atmung eines Lebewesens.

Es ist vorgesehen, dass zumindest die Gasströmung der Atmung in Form von Druck und/oder Fluss durch das System simuliert wird. Darunter fällt eine Strömung von Gas durch eine Atmungsöffnung in das System, um die Inspiration eines Lebewesens zu simulieren und eine Strömung von Gas durch eine Atmungsöffnung aus dem System, um die Exspiration zu simulieren. In manchen Ausführungsformen ist vorgesehen, dass auch der physiologische Gastausch in der Lunge durch das System simuliert wird. Zumindest wird dabei simuliert, dass Sauerstoff aus dem Gas aufgenommen wird und CO2 an das Gas abgegeben wird.

Das System ist darauf ausgelegt, eine Atmung anhand der mathematischen Simulation vorzugeben und/oder die Simulation anhand von äußeren Quellen, beispielsweise einem verbundenen Beatmungsgerät, anzupassen.

Das erfinderische System ist dazu eingerichtet und ausgebildet mit jedweden Arten von Geräten, welche mit einem Gasstrom interagieren, verbunden zu werden.

Dazu zählen beispielsweise jegliche Beatmungsgeräte nach dem Stand der Technik und/oder Diagnosegeräte zur Analyse der Atmung/des Atemgases. Auch andere Gasquellen, beispielsweise eine manuelle Beatmung über Mund-zu-Mund-Beatmung und/oder einen Beutel sowie Druckgasflaschen bzw. Pressluft, wie beim Atemschutz und/oder Tauchern üblich, können mit dem System verbunden werden.

In manchen Ausführungsformen ist daran gedacht, dass, gegebenenfalls über einen zusätzlichen Adapter (etwa ein künstlicher Kopf), auch Beatmungsmasken und/oder Patienteninterfaces mit dem System verbunden werden. Um etwa Einflüsse und/oder Eigenschaften einer Maske bestimmen zu können, kann vorgesehen sein, dass weitere Sensoren im Bereich der Maske, etwa im künstlichen Kopf, angeordnet sind. Es kann alternativ oder ergänzend auch vorgesehen sein, dass eine Maske zusätzlich simuliert wird. Etwa kann vorgesehen sein, dass eine Maskenleckage simuliert wird. Die Simulation einer Maske erfolgt beispielsweise in der mathematischen Simulation, sodass keine zusätzlichen Modifikationen an dem Gasmodul nötig sind, um beispielsweise eine Leckage zu simulieren. Alternativ oder ergänzend kann auch ein steuerbares Ventil vorgesehen sein, durch welches Atemgas aus dem Gasmodul entweichen kann, ohne in Richtung eines angeschlossenen Beatmungsgerätes zu strömen, um eine Leckage auch physikalisch zu simulieren.

Es kann auch vorgesehen sein, dass eine Beatmungsmaske und/oder weitere Patienteninterfaces über die mathematische Simulation simuliert werden. Im Ergebnis wirkt die mathematische Simulation dann so, als ob das simulierte Lebewesen, beispielsweise ein menschlicher Patient, ein Patienteninterface nutzt. Über das Gasmodul kann entsprechend auch eine physikalische Umsetzung der mathematischen Simulation wiedergegeben werden. Wird beispielsweise ein Beatmungsgerät mit dem Gasmodul verbunden, kann das Gasmodul auf das Beatmungsgerät so wirken, als ob ein Lebewesen mit Patienteninterface mit dem Beatmungsgerät verbunden ist. Zu dem Patienteninterface können beispielsweise Parameter wie Leckage, Volumen (zum Beispiel Maskenvolumen) und/oder pneumatische Widerstände mit einbezogen werden. Zudem kann auch vorgesehen sein, dass ein zusätzlicher Filter, beispielsweise ein HME Filter, am Patienteninterface mit in die mathematische Simulation einbezogen wird. Etwa können das Volumen und der pneumatische Widerstand des Filters als Parameter in die Simulation einfließen. Ein weiterer Parameter kann auch die O2/CO2-Konzentration oder die allgemeine Gasmischung in der Maske sein, beispielsweise Zwecks O2/CO2-Auswaschung oder O2/CO2-Akkumulation in der Maske. Ein weiterer Parameter kann auch das Totraumvolumen der Maske sein.

Es kann auch vorgesehen sein, dass das System zur Diagnose eines Patienten genutzt werden kann. Beispielsweise kann die Atmung des Patienten über das Gasmodul über Sensoren gemessen werden, wobei das Steuermodul dazu eingerichtet ist über die mathematische Simulation Aspekte der Atmung des Patienten zu simulieren. Beispielsweise kann vorgesehen sein, dass über die mathematische Simulation Werte/Parameter/Situationen des Patienten aus den gemessenen Werten simuliert werden, welche über die reinen Messwerte hinausgehen. Beispielsweise kann eine genaue Situation in der Lunge des Patienten über die Messwerte mathematisch simuliert werden, wobei die mathematische Simulation einen zumindest annähernden Zustand wiedergibt.

Das System ist dazu eingerichtet und ausgebildet, die Atmung eines Lebewesens, insbesondere von Menschen, zu simulieren.

Es ist vorgesehen, dass die Gasströmung, welche beispielsweise über einen Anschluss, über welchen das System mit externen Geräten bzw. Mitteln verbunden und/oder interagieren kann, aus dem System geleitet wird, die Atmung eines Lebewesens nachstellt.

In manchen Ausführungsformen wird insbesondere der durch das Lebewesen erzeugte Druck simuliert. Dabei wird über die mathematische Simulation ein Druckverlauf berechnet, welcher der Lungenkontraktion und Lungenexpansion während der Atmung entspricht. Dies entspricht weitestgehend der natürlichen Atmung eines Menschen/Säugetiers, welches bei der Ausatmung einen positiven Druck in der Lunge erzeugt, durch welchen das Gas aus der Lunge gedrückt wird und bei der Einatmung über einen Unterdruck Gas in die Lunge saugt. Hierbei ist zu beachten, dass so der durch die Lunge bzw. das Lebewesen erzeugte Druck beschrieben wird. Ist beispielsweise ein Beatmungsgerät mit dem Gasmodul verbunden, welches zusätzlich einen Druck erzeugt, so wird der durch die Simulation erzeugte Druck zusätzlich mit dem Druck des Beatmungsgerätes beaufschlagt. So kann es auch vorkommen, dass innerhalb des Gasmoduls konstant ein positiver Druck gemessen wird. Durch die Simulation der Atmung schwankt, je nach Kompensation durch das Beatmungsgerät, der Druck um den vom Beatmungsgerät erzeugten Druck.

In manchen Ausführungsformen ist das System mit Eingabemodulen, wie beispielsweise einem Computer, Laptop, Tablet, mobilem Endgerät (Handy) verbunden. Diese Eingabemodule können beispielsweise auch zu Erzeugung einer Augmented Reality, also einer erweiterten Realität, dienen. Beispielsweise werden auf einem Tablet dann die durch die mathematische Simulation errechneten Werte angezeigt und/oder entsprechende Informationen angezeigt und gegebenenfalls mit Körperbereichen eines Patienten verknüpft.

Die Erfindung betrifft auch ein Verfahren zur Simulation der Atmung eines Lebewesens, insbesondere von Menschen.

In einer beispielhaften Variante umfasst das Verfahren einen Verfahrensschritt der Eingabe von Daten zur mathematischen Simulation der Atmung.

Die Eingabe von Daten kann dabei beispielsweise eine Eingabe von Vorgaben sein, zum Beispiel Druck, Fluss, Volumen, Frequenz, Gaszusammensetzung und/oder zu simulierenden Atemsituationen und/oder Atemproblemen sein. Die Daten können alternativ oder ergänzend auch Sensordatendaten sein.

In einem Verfahrensschritt ist die mathematische Simulation der Atmung anhand der eingegebenen Daten vorgesehen.

Dabei wird, beispielsweise computergestützt, der zeitliche Verlauf der Atmung sowie die zugehörigen Parameter anhand der Eingaben berechnet. In manchen Ausführungsformen umfasst die mathematische Simulation eine Berechnung bzw. Simulation von zusätzlichen Atmungsparametern und/oder der Situation in der Lunge und/oder Luftröhre eines realen Patienten anhand von erfassten Sensorwerten.

Die mathematische Simulation wird in einem folgenden Verfahrensschritt in Steuerbefehle zur Steuerung eines Gasmoduls übersetzt. Diese Steuerbefehle werden beispielsweise an eine Steuereinheit übermittelt. Anhand der Steuerbefehle wird das Gasmodul angesteuert. In manchen Ausführungsformen ist vorgesehen, dass die Steuerbefehle so umgesetzt werden, dass das Gasmodul die mathematische Simulation in eine physikalische Simulation umsetzt.

**In** manchen Ausführungsformen ist vorgesehen, dass die Steuerbefehle, welche aus der mathematischen Simulation abgeleitet werden, an ein Beatmungsgerät übertragen werden, um die Arbeitsweise des Beatmungsgerätes anzupassen.

Ein weiterer Verfahrensschritt umfasst die Aufnahme, Aufbereitung und/oder Auswertung von Werten bezüglich der physikalisch simulierten Atmung und/oder der Atmung eines realen Lebewesens. Die Werte können dann ausgewertet werden und/oder in die mathematische Simulation eingebracht werden. Beispielswiese werden die von den Sensoren erfassten bzw. aufgenommenen Werte ausgewertet hinsichtlich der korrekten physikalischen Simulation.

Alternativ oder ergänzend wird auf Basis der erfassten Werte und/oder der Auswertung die mathematische Simulation, bevorzugt automatisch, angepasst. In manchen Ausführungsformen bilden die von den Sensoren erfassten Werte auch die Basis der mathematischen Simulation, beispielsweise um ergänzende Werte, Informationen und/oder Daten des Patienten zu simulieren. Die Werte zur Atmung können in manchen Ausführungsformen auch durch Werte, Daten und/oder Informationen zu weiteren Körperfunktionen und/oder Eigenschaften des Lebewesens ergänzt werden.

In einer beispielhaften Ausführungsform des Verfahrens werden zu bzw. vor Beginn der Simulation Daten, Informationen und/oder Werte eingegeben, welche als Vorgaben zur mathematischen Simulation der Atmung eines Lebewesens, bevorzugt eines Menschen, dienen. Anhand dieser Vorgaben wird in der mathematischen Simulation die Atmung des Lebewesens berechnet bzw. simuliert. Die Vorgaben zur mathematischen Simulation umfassen dabei beispielsweise zumindest Werte zu Druck und Fluss der Atmung. Beispielsweise können maximale und minimale Drücke sowie Flüsse angegeben werden, welche dann bei der Simulation der Inspiration und Exspiration miteinbezogen werden. Auch weitere Vorgaben, beispielsweise bezüglich der Atemfrequenz, Gaszusammensetzung, Gastemperatur, Gasfeuchtigkeit, Atemzugsvolumen und/oder Gasaustausch in der Lunge, können in manchen Ausführungsformen eingegeben werden.

Es kann auch vorgegeben sein, bestimmte Bereiche für die Vorgaben zu definieren, beispielsweise in Form eines Maximal- und Minimalwertes und/oder auch in Form eines Mittelwertes und/oder eines Idealwertes.

Die mathematische Simulation kann beispielsweise auch einen Zufallsgenerator umfassen, welcher für eine gewisse Ungleichmäßigkeit bei der simulierten Atmung sorgt. Beispielsweise kann so eine realistischere Atmung simuliert werden. Der Zufallsgenerator kann zum Beispiel zufällig die Werte für Druck, Fluss, Gaszusammensetzung und/oder Atemzugsvolumen um einen Mittelwert schwanken lassen.

Es kann auch vorgesehen sein, dass Vorgaben zu Atemereignissen bzw. Atemsituationen eingegeben werden und diese mit in die mathematische Simulation aufgenommen werden. Beispielsweise kann eine Anzahl, Dauer und/oder Zeitpunkt für das Auftreten verschiedener Atmungssituationen vorgegeben werden. Auch Vorgaben bezüglich des Lebewesens, wie Gewicht, Alter, Größe, Geschlecht, Vorerkrankungen, etc., können als Vorgaben vorgesehen sein.

Die mathematische Simulation dient im Laufe des Verfahrens als Grundlage zur Steuerung eines Gasmoduls. Beispielsweise wird die mathematische Simulation in Steuerbefehle umgesetzt, anhand derer das Gasmodul gesteuert wird. Es ist beispielsweise vorgesehen, dass gleichzeitig mit der mathematischen Simulation der derzeitige mathematische Simulationszustand unmittelbar in einen Steuerbefehl umgesetzt wird, sodass das Gasmodul im Zeitverlauf immer den aktuellen mathematischen Simulationszustand darstellt. Beispielsweise ist vorgesehen, dass das Gasmodul auf ein angeschlossenes Beatmungsgerät wie ein reales Lebewesen wirkt, insbesondere hinsichtlich der Atmung.

Gleichzeitig werden Messwerte bezüglich der durch das Gasmodul, gegebenenfalls im Zusammenspiel mit einem angeschlossenen Beatmungsgerät, erzeugten physikalisch simulierten Atmung aufgenommen. Es kann vorgesehen sein, dass diese Messwerte unmittelbar ausgewertet werden. Die Auswertung umfasst zum Beispiel eine Analyse, ob die durch die mathematische Simulation vorgesehene Atmung auch richtig wiedergegeben wird. Auch kann über die Analyse der Messwerte analysiert werden, ob und/oder wie ein Beatmungsgerät auf die simulierte Atmung reagiert. Es kann zusätzlich vorgesehen sein, dass das Ergebnis der Auswertung unmittelbar in die mathematische Simulation mit einbezogen wird. Beispielsweise kann vorgesehen sein, dass über die mathematische Simulation auf eine Arbeitsweise des Beatmungsgerätes reagiert wird.

In manchen Ausführungsformen kann alternativ oder ergänzend vorgesehen sein, dass Messwerte der Atmung einer realen Person als Vorgaben zur mathematischen Simulation dienen. Es kann dabei vorgesehen sein, dass die Messwerte der realen Person als Grundlage einer mathematischen Simulation genutzt werden, über welche ergänzende Informationen, Werte und/oder Daten der Atmung der realen Person simuliert werden. Es kann vorgesehen sein, dass beispielsweise anhand der ergänzenden Informationen/Daten/Werte ein Beatmungsgerät (als Gasmodul) angesteuert wird und/oder diese ergänzenden Informationen/Daten/Werte an das Beatmungsgerät weitergeleitet werden und das Beatmungsgerät die Beatmung der realen Person gegebenenfalls anpassen kann. Beispielsweise kann anhand von Messwerten die Situation in der Lunge und/oder Luftröhre der realen Person mathematisch simuliert werden, wobei die ergänzenden Informationen/Werte/Daten messtechnisch nicht oder nur unter größerem Aufwand erfassbar sind.

In manchen Ausführungsformen kann vorgesehen sein, dass über die mathematische Simulation ein Husten simuliert wird. Eine solche Simulation kann beispielsweise über Ausführungsformen des Gasmoduls mit zwei Gebläsen in eine physikalische Simulation umgesetzt werden.

Auch kann vorgesehen sein, dass verschiedene Größen und Arten an Lebewesen simuliert werden können. Je nach Größe des Lebewesens bzw. des Lungenvolumens und Gasflusses sind gegebenenfalls entsprechende Änderungen an dem Gasmodul vorgenommen werden. Beispielsweise kann vorgesehen sein, dass ein großes Atemzugsvolumen durch eine Vielzahl an Gebläsen oder besonders große und/oder leistungsfähige Gebläse erreicht wird.

In manchen Ausführungsformen kann vorgesehen sein, dass zumindest zwei Gebläse in Serie geschaltet werden. Dabei können zumindest zwei Gebläse eine entgegengesetzte Förderrichtung aufweisen. Über eine solche Anordnung können mehrere fortgeschrittene Simulationen angeboten werden. Mit zwei gegeneinander wirkenden Gebläsen kann auf schneller wechselnde Beatmungsdrücke schnell reagiert werden.

In den bespielhaften Ausführungsformen, welche im Rahmen der Figuren diskutiert werden, ist beispielhaft ein Beatmungsgerät mit dem System verbunden. Die Verbindung ist rein exemplarisch zu verstehen und schließt eine Verbindung des Systems mit anderen Geräten und/oder Mitteln nicht aus.

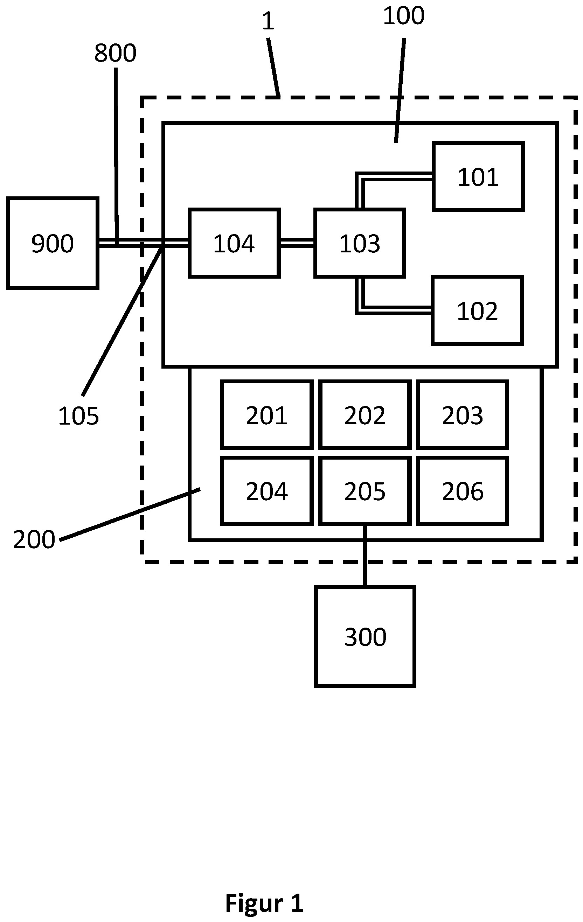

In Figur 1 ist eine beispielhafte Ausführungsform des Systems 1 schematisch dargestellt. Das System 1 ist dazu eingerichtet und ausgebildet die Atmung eines Lebewesens, beispielsweise eines Menschen, zu simulieren. Die Simulation der Atmung umfasst dabei zwei Simulationsteile. In dem ersten Simulationsteil wird die Atmung des Lebewesens in dem Steuermodul 200 mathematisch bzw. rechnerisch simuliert. Für den zweiten Simulationsschritt wird das Gasmodul 100 durch das Steuermodul 200 auf Basis der mathematischen Simulation angesteuert. In der dargestellten Ausführungsform ist beispielhaft vorgesehen, dass die mathematische Simulation in eine physikalische Simulation durch das Gasmodul 100 umgesetzt wird.

Beispielhaft ist das System 1 über eine Verbindung mit einem Beatmungsgerät 900 verbunden. Bei dem Beatmungsgerät 900 kann es sich um jede Art von Beatmungsgerät nach dem Stand der Technik handeln. Neben einem Beatmungsgerät kann hier auch ganz allgemein eine Beatmung angesetzt werden, also eine maschinelle und/oder manuelle Beatmung (z.B. Mund-zu-Mund, Beatmungsbeutel). Auch eine Beatmung über Pressluftflaschen, wie beim Atemschutz und/oder Tauchern üblich, kann mit dem System 1 verbunden werden. Um beispielsweise die Funktionsfähigkeit von Diagnosegeräten zu testen, können in manchen Ausführungsformen auch entsprechende Diagnosegeräte mit dem System 1 verbunden werden. Über die Verbindung 800 kann eine gasleitende Verbindung zwischen dem System 1 und dem Beatmungsgerät 900 hergestellt werden, beispielsweise über den Anschluss 105 des Gasmoduls 100.

Das Gasmodul 100 des Systems 1 umfasst beispielhaft eine Exspirationseinheit 101, eine Inspirationseinheit 102, ein optionales Ventil 103 zur Steuerung bzw. Umschaltung zwischen Inspiration und Exspiration, sowie Sensorik 104 zur Bestimmung der Atmungsparameter innerhalb des Gasmoduls 100. Die Exspirationseinheit 101, beispielsweise als Druckgasquelle und/oder Gebläse ausgebildet, ist dazu eingerichtet die Exspiration zu simulieren, also einen Gasstrom zu erzeugen, welcher durch den Anschluss 105 aus dem System entweichen kann und zumindest in Druck- und/oder Flussprofil der Atmung eines Lebewesens entspricht. Beispielsweise wird über die Exspirationseinheit 101 und die Inspirationseinheit 102 ein Druck erzeugt, welcher dem durch ein Lebewesen in den Lungen erzeugten Druck entspricht.

Die Inspirationseinheit 102 ist dazu eingerichtet eine Inspiration eines Lebewesens zu simulieren. Die Inspirationseinheit 102 kann dazu beispielsweise ein Gebläse und/oder eine Vakuumpumpe und/oder andere Einrichtungen umfassen, mit welcher auf der Seite des Anschlusses 105 ein Unterdruck erzeugt werden kann bzw. Gas aus dem System gezogen wird.

An dieser Stelle ist anzumerken, dass Gebläse zwar in eine Richtung einen Druck aufbauen können, durch ein Gebläse aber durchaus in zwei Richtungen der Gasfluss erfolgen kann. Damit über ein Gebläse ein Gasfluss in die Druckrichtung erfolgt, muss das Gebläse den Gegendruck, erzeugt beispielsweise durch ein Beatmungsgerät, überwinden, also einen höheren Druck bereitstellen als das Beatmungsgerät.

In manchen Ausführungsformen ist die Inspirationseinheit 102 so eingerichtet, dass beispielsweise ein Ventil geöffnet wird, optional in Zusammenhang mit einem pneumatischen Widerstand und/oder einem variablen Volumen wie z.B. einem Ballon, durch welche ein Gas durch den Anschluss 105 in das System strömen kann. Durch den pneumatischen Widerstand bzw. das Volumen kann das Befüllen der Lunge mit Gas durch eine externe Quelle, beispielsweise eine Beatmung/ein Beatmungsgerät 900, simuliert werden. In manchen Ausführungsformen ist die Inspirationseinheit 102 so schaltbar, dass sowohl ein zumindest teilweise aktives Einatmen als auch ein passives, also von einer externen Quelle vorgegebenes Einatmen simuliert wird.

In manchen Ausführungsformen ist vorgesehen, dass die Exspirationseinheit 101 und die Inspirationseinheit 102 als eine kombinierte Einheit ausgebildet sind, welche beide Funktionen, Simulation der Inspiration und Exspiration, umfasst. In manchen Ausführungsformen sind die Inspirationseinheit 102 und/oder die Exspirationseinheit 101 und/oder die kombinierte Einheit dazu ausgebildet eine bestimmte Gasmischung zu erzeugen.

Beispielsweise ist vorgesehen, dass durch die Exspirationseinheit 101 neben einer Simulation der Gasströmung auch die Aufnahme von Sauerstoff und Abgabe von CO2 in der Lunge simuliert wird. Dabei wird beispielsweise ein, optional definierbares, Gasgemisch durch die Exspirationseinheit 101 erzeugt bzw. gemischt.

Ein Umschalten zwischen simulierter Inspiration und Exspiration wird beispielsweise durch ein optionales Ventil 103 realisiert. Optional ist das Ventil 103 so eingerichtet, dass ein stufenloses Umschalten zwischen Exspirationseinheit 101 und Inspirationseinheit 102 möglich ist. Alternativ ist auch eine Regelung nur über die Inspirationseinheit 102 und die Exspirationseinheit 101 möglich, ohne dass das eine extra Ventil 103 verwendet werden muss.

In manchen Formen der Ansteuerung ist es vorgesehen, dass ein Übergang von Inspiration zu Exspiration bzw. umgekehrt, dadurch simuliert wird, dass das Ventil 103 entsprechend stufenlos umschaltet. So kann zum Beispiel ein nach und nach abnehmender Gasfluss zum Ende der Exspiration simuliert werden, und durch Variation der Schaltgeschwindigkeit der im Vergleich zur Abnahme des Gasflusses der Exspiration, schnell zunehmende Gasfluss der Inspiration nachgestellt werden. Hierbei ist gegebenenfalls zu beachten, dass die Regelung durch ein häufiges hin und herschalten bei Flüssen im Bereich von Null beeinträchtigt werden kann.

Die Sensorik 104 ist dazu eingerichtet und ausgebildet, Messwerte des Gases im Gasmodul 100 zu erfassen. Die Messwerte betreffen beispielsweise Druck, Fluss, Temperatur, Feuchtigkeit und/oder Gaszusammensetzung. Die Sensorik 104 ist beispielsweise zwischen der Exspirationseinheit 101 bzw. Inspirationseinheit 102 und dem Anschluss 105 angeordnet. Die Sensorik 104 ist so angeordnet, dass durch die erfassten Messwerte beispielsweise die Gasparameter entsprechend einer Lunge und/oder Luftröhre des simulierten Lebewesens wiedergegeben werden. Über die Sensorik 104 kann beispielsweise gemessen werden, ob die Atmung entsprechend der Vorgaben, beispielsweise der mathematischen Simulation, simuliert wird. Auch kann der Einfluss des Beatmungsgerätes 900 bzw. anderer über den Anschluss 105 verbundener Geräte und/oder Mittel auf die Atmung erfasst werden.

Zur Steuerung des Gasmoduls 100 umfasst das Steuermodul 200 eine Steuereinheit 201, welche dazu ausgebildet ist, zumindest die Exspirationseinheit 101 und die Inspirationseinheit 102 zu steuern. Ist ein Ventil 103 zum Umschalten zwischen Exspiration und Inspiration vorgesehen, so wird dieses auch über die Steuereinheit 201 gesteuert. Die Steuereinheit 201 ist ferner dazu eingerichtet und ausgebildet auf Grundlage der mathematischen Simulation der Atmung das Gasmodul 100 anzusteuern.

Das Steuermodul 200 umfasst zur mathematischen Simulation der Atmung eine Simulationseinheit 202. Die Simulationseinheit 202 ist dazu eingerichtet und ausgebildet die Atmung eines Lebewesens anhand von Vorgaben und/oder Eingaben mathematisch zu simulieren. In manchen Ausführungsformen ist vorgesehen, dass die Simulationseinheit 202 anhand der mathematischen Simulation der Atmung Steuersignale zu erzeugen, welche von der Steuereinheit 201 verwendet werden, um das Gasmodul 100 entsprechend anzusteuern. Beispielhaft ist vorgesehen, dass die Simulationseinheit 202 in einem ersten Simulationsteil die Atmung mathematisch simuliert, wobei die mathematische Simulation durch die Steuereinheit 201 und das Gasmodul 100 in eine physikalische Simulation der Atmung umgesetzt wird.

Das Steuermodul 200 des Systems 1 umfasst zudem beispielhaft eine Sensoreinheit 204, eine Auswerteeinheit 203, eine Eingabeeinheit 205 sowie eine Speichereinheit 206. Die Sensoreinheit 204 ist dazu eingerichtet und ausgebildet die über die Sensorik 104 erfassten Messwerte aufzunehmen und gegebenenfalls aufzubereiten. Die Auswerteeinheit 204 ist dazu eingerichtet und ausgebildet, die von der Sensoreinheit 204 aufgenommenen und optional aufbereiteten Messwerte auszuwerten und/oder zu analysieren. Beispielsweise kann vorgesehen sein, dass die Auswerteeinheit 204 die Messwerte dahingehend analysiert, ob die durch die mathematische Simulation vorgegebene Steuerung des Gasmoduls 100 korrekt erfolgt, beispielsweise die gewünschten Drücke, Flüsse und/oder Volumina erzeugt werden. Die Ergebnisse der Analyse und/oder Auswertung werden beispielsweise über die Eingabeeinheit 205 zur Simulationseinheit 202 weitergeleitet. Über die Simulationseinheit 202 können die Analyseergebnisse und/oder auch die Messwerte selbst mit in die mathematische Simulation einbezogen werden, welche die Grundlage der Steuerung des Gasmoduls 100 bilden.

Beispielhaft umfasst das Steuermodul 200 eine Speichereinheit 206. In der Speichereinheit 206 können Messwerte, Analysen und/oder Auswertungen zumindest zwischengespeichert werden. In manchen Ausführungsformen des Systems 1 ist vorgesehen, dass sowohl die mathematische Simulation als auch die durch die Sensorik 104 erfassten Messwerte in der Speichereinheit 206 abgespeichert werden, beispielsweise für einen späteren Vergleich zwischen mathematischer und physikalischer Simulation.

Die Eingabeeinheit 205 dient bespielhaft als Schnittstelle, über welche Daten, Werte und/oder Informationen in das System 1, insbesondere in das Steuermodul 200, eingegeben werden können. In manchen Ausführungsformen erfolgt auch eine systeminterne Eingabe von Daten, Werten und/oder Informationen über die Eingabeeinheit 205, beispielsweise aus der Auswerteeinheit 204, an die Simulationseinheit 202. Es ist auch angedacht, dass die Eingabeeinheit 205 dazu eingerichtet und ausgebildet ist, Daten, Werte und/oder Informationen an ein externes Gerät weiterzugeben. Beispielsweise kann das Eingabemodul 300 als Computer, Notebook, Smartphone und/oder Tablet ausgebildet sein und dazu eingerichtet sein, Werte, Daten und/oder Informationen des Systems 1 anzuzeigen und gegebenenfalls zu speichern.

Das Eingabemodul 300 ist insbesondere dazu eingerichtet, Vorgaben und/oder Einstellungen bezüglich der Simulation der Atmung in das System 1 einzugeben. Die Simulationseinheit 202 ist beispielsweise dazu eingerichtet und ausgebildet, anhand der Vorgaben und/oder Einstellungen die Atmung des Lebewesens mathematisch zu simulieren. Die Vorgaben und/oder Einstellungen umfassen beispielsweise aber nicht ausschließend Druck, Fluss, Lungenvolumen, Gaszusammensetzung, Atemfrequenz, Atemzugsvolumen, Art des Lebewesens, Alter, Gewicht, Krankheiten (insbesondere respiratorische Krankheiten), Gasaustausch, Atemprobleme. In manchen Ausführungsformen ist vorgesehen, dass das Eingabemodul 300 über eine Eingabemaske verfügt, über welche Einstellungen bezüglich der Simulation der Atmung eingegeben werden, welche an das Steuermodul 200 übermittelt werden. In manchen Ausführungsformen sind in der Speichereinheit 206 eine Vielzahl an Simulationsvorlagen und/oder Simulationsabläufen hinterlegt, auf welche über das Eingabemodul 300 zugegriffen werden kann.

In manchen Ausführungsformen ist es vorgesehen, dass eine Anzeige der aktuellen Simulation der Atmung, beispielsweise in Form von Werten und/oder Graphen, über das Eingabemodul 300 möglich ist.

In manchen Ausführungsformen ist vorgesehen, dass mehrere Eingabemodule 300 mit der Eingabeeinheit 205 des Steuermoduls 200 verbunden werden können. Beispielsweise ist auch eine Verbindung mit weiteren Sensoren, Aktuatoren, einer virtuellen Realität und/oder Patientensimulatoren möglich. Es ist zudem optional vorgesehen, dass Eingabemodule 300 auch zur Ausgabe von Werten, Daten, Informationen, Anzeigen, etc. dienen können.

In manchen Ausführungsformen umfasst die Simulation der Atmung, insbesondere die mathematische Simulation, auch die Simulation weiterer physiologischer Parameter. Die Simulation weiterer physiologischer Parameter kann beispielsweise den Blutkreislauf und/oder die Körpertemperatur umfassen. Auch weitere physiologische Parameter bzw. Abläufe können optional zumindest in die mathematische Simulation miteinbezogen werden und/oder simuliert werden. Die Simulationseinheit 202 ist beispielsweise dazu eingerichtet und ausgebildet, die Auswirkung der weiteren physiologischen Parameter auf die Simulation der Atmung mit in die mathematische Simulation einfließen zu lassen und entsprechende Steuersignale für die Steuereinheit 201 zu erzeugen.

Es kann weiter vorgesehen sein, dass alternativ oder ergänzend ein Eingabemodul 300, beispielsweise Mittel zur Eingabe und Anzeige von Daten, Werten und Informationen umfassend, in das System 100 integriert ist. Mittel zur Eingabe können beispielsweise eine Tastatur und/oder Maus sein. Mittel zur Anzeige kann beispielsweise ein Bildschirm sein. Es kann auch eine kombinierte Mittel zur Eingabe und Anzeige vorgesehen sein, beispielsweise ein berührungsempfindlicher Bildschirm (Touchscreen).

Für die in Figur 1 beispielhaft dargestellte Ausführungsform des Systems 1 ist vorgesehen, dass die Simulation der Atmung vollständig über das System 1 stattfindet. Die Simulation umfasst dabei den ersten Simulationsteil (mathematische Simulation) und den zweiten Simulationsteil (Steuerung des Gasmoduls 100 zur physikalischen Simulation der Atmung).

Über den Anschluss 105 des Gasmoduls 100 ist beispielhaft ein Beatmungsgerät 900 mit dem System 1 bzw. dem Gasmodul 100 verbunden. Dem Beatmungsgerät 900 gegenüber simuliert das System 1 beispielsweise die Atmung eines Lebewesens.

Das System 1 ist so eingerichtet und ausgebildet, dass eine Vielzahl verschiedener Atmungssituationen bzw. Atemereignisse simuliert werden kann.

Beispielsweise kann im eingestellten Simulationsverlauf vorgesehen sein, dass eine Atemwegsblockade simuliert wird. Das Beatmungsgerät 900 ist beispielsweise so eingerichtet und ausgebildet, dass auf eine Atemwegsblockade mit einer Druckerhöhung bei der Beatmung reagiert wird, bis die Apnoe behoben wird. Die Sensorik 104 erfasst dabei diese Druckerhöhung durch das Beatmungsgerät 900 und leitet die Messwerte an das Steuermodul 200 weiter. Über die Auswerteeinheit 204 und/oder die Simulationseinheit 202 wird die erfolgte Druckerhöhung durch das Beatmungsgerät 900 analysiert und/oder ausgewertet, ob die Druckerhöhung ausreichend ist, um die Atemwegsblockade zu behandeln bzw. zu beheben. Ergibt die Analyse und/oder Auswertung, dass die Druckerhöhung ausreichend zur Lösung der Atemwegsblockade ist, beispielsweise anhand eines Vergleichs mit gespeicherten Vorgaben, so passt die Simulationseinheit 202 die mathematische Simulation und die Steuersignale für die physikalische Simulation entsprechend an.

Für die physikalische Simulation weiterer Aspekte der Atmung bzw. der Lungenfunktion eines Lebewesens kann das System 1 zusätzlich auch einen Atemgasanfeuchter und/oder eine Atemgasheizung umfassen.

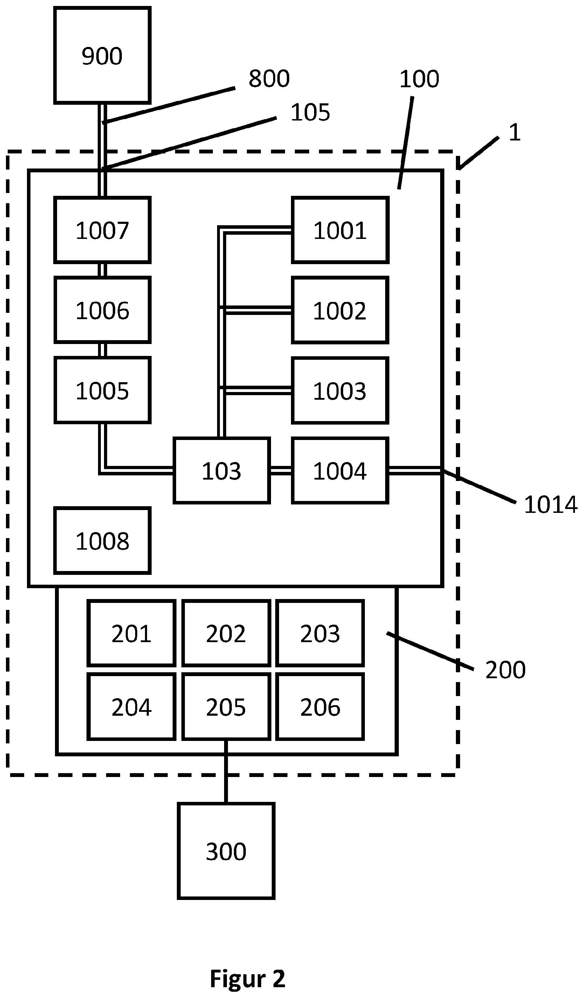

In Figur 2 ist eine weitere beispielhafte Ausführungsform des Systems 1 schematisch dargestellt. Zur physikalischen Simulation der Exspiration sind die Gasquellen 1001, 1002, 1003 vorgesehen, welche zusammen die Exspirationseinheit 101 bilden (vgl. Figur 1). Beispielsweise sind zusammen mit den Gasquellen 1001, 1002, 1003 jeweils entsprechende Ventile und Drucksensoren angeordnet (nicht explizit dargestellt). Beispielsweise kann es sich bei den Ventilen, welche den Gasquellen 1001, 1002, 1003 zugeordnet sind um "Open-Close"-Ventile handeln, also Ventile die entweder geöffnet oder geschlossen sind. Über eine gepulste Ansteuerung, also das Öffnen oder Schließen der Ventile über einen definierten Zeitraum, kann eine genaue Einstellung der Gaszusammensetzung erreicht werden. Werden die Ventile pro Puls für einen definierten Zeitraum, bzw. für die Pulslänge, geöffnet, strömt ein definiertes Volumen durch das Ventil. Durch Festlegen der Pulslängen und/oder Pulsanzahlen und/oder Pulsfrequenz kann so genau definiert ein Gasvolumen durch das Ventil strömen was zu einer hohen Genauigkeit beim Einstellen der Gaszusammensetzung aus den Gasquellen 1001, 1002, 1003 genutzt werden kann. Alterativ oder ergänzend kann vorgesehen sein, dass die Ventile der Gasquellen 1001, 1002, 1003 Proportionalventile sind.

Über die Gasquellen 1001, 1002, 1003 kann zur physikalischen Simulation der Exspiration ein Gasgemisch zur Verfügung gestellt werden, welches dem Gasgemisch entspricht, das durch das simulierte Lebewesen ausgeatmet wird. Beispielsweise kann so die Sauerstoffaufnahme aus der Atemluft und die CO2-Abgabe an die Atemluft in der Lunge simuliert werden. Beispielsweise ist die Gasquelle 1001 als CO2-Quelle ausgebildet, die Gasquelle 1002 als Sauerstoffquelle und die Gasquelle 1003 als Stickstoffquelle. Über die entsprechenden Partialdrücke der Gasquellen 1001, 1002, 1003 kann beispielsweise das Gasgemisch gezielt eingestellt werden.

Zur physikalischen Simulation der Inspiration umfasst das Gasmodul 100 beispielhaft eine Vakuumpumpe 1004 zusammen mit Ventil und Drucksensor als Inspirationseinheit 102 (vgl. Figur 1). Statt einer Vakuumpumpe können alternativ oder ergänzend auch andere Mittel eingesetzt werden, welche die Generation eines Unterdrucks ermöglichen In manchen Ausführungsformen wirken die Gasquellen 1001, 1002, 1003 und die Vakuumpupe 1004 zusammen als eine kombinierte Inspirations- und Exspirationseinheit. Durch eine entsprechende Steuerung der Gasquellen, Vakuumpumpe und Ventile wird so im Gasmodul 100 die Atmung physikalisch simuliert.

Optional ist zusätzlich ein Ventil 103 vorgesehen, welches angesteuert wird, um zwischen Inspiration und Exspiration umzuschalten. Dazu ist angedacht, dass das Ventil 103 so angeordnet und ausgebildet ist, dass eine stufenlose Schaltung zwischen den Gasquellen 1001, 1002, 1003 einerseits und der Vakuumpumpe 1004 andererseits ermöglicht wird. Beispielsweise wird zu Beginn der simulierten Exspiration das Ventil 103 so geschaltet, dass zumindest teilweise, in manchen Ausführungsformen hauptsächlich oder ausschließlich, Gas aus den Gasquellen durch die Leitungen zum Anschluss 105 geleitet wird. Zur Simulation der Inspiration wird hingegen beispielsweise das Ventil 103 so geschaltet, dass Gas vom Anschluss 105 in Richtung Vakuumpumpe 1004 beziehungsweise Auslass 1014 geleitet wird. Die Gasquellen 1001, 1002, 1003 können beispielsweise im Gasmodul 100 angeordnete Gasflaschen bzw. Druckgasanschlüsse sein und/oder Anschlüsse für externe Gasquellen wie z.B. Gasflaschen und/oder Druckgasleitungen sein. Alternativ oder ergänzend kann auch vorgesehen sein, dass die Vakuumpumpe 1004 im Gasmodul 100 angeordnet ist, sondern, dass eine extern angeordnete Vakuumpumpe (bzw. Unterdruckquelle) mit dem Gasmodul 100 verbunden wird. Sind die Ventile der Gasquellen 1001, 1002, 1003 als "Open-Close"-Ventile vorgesehen, so kann ein weiteres "Open-Close"-Ventil vor der Vakuumpumpe 1004 angeordnet sein. Beispielsweise kann dann das Ventil 103 entfallen. Das Umschalten zwischen Inspiration und Exspiration erfolgt dann beispielsweise über das Schalten der jeweiligen Ventile erreicht werden.

Die Sensorik 104 (siehe Figur 1) umfasst in der in Figur 2 dargestellten Ausführungsform einen Drucksensor 1005, einen Flusssensor 1006, einen Temperatursensor 1007 sowie einen zweiten Drucksensor 1008. Zudem können für die Sensorik 104 weitere Sensoren vorgesehen sein, beispielsweise zur Erfassung der Gaszusammensetzung und/oder der Gasfeuchtigkeit. Der Drucksensor 1008 ist dazu angeordnet und ausgebildet, den Luftdruck der Umgebungsluft zu messen. Über den Drucksensor 1005, den Flusssensor 1006 sowie den Temperatursensor 1007 werden Druck, Fluss und Temperatur des Gases der gesamten simulierten Atmung (inklusive etwaiger extern Einflüsse) erfasst, welche, auf ein Lebewesen übertragen, den Werten in Lunge und/oder Luftröhre entsprechen. Wird das Gas beispielsweise mit einem Druck durch das Beatmungsgerätes 900 beaufschlagt, so wird durch den Druckmesser 1005 der Druck des Gases bestimmt, welcher sich aus der simulierten Atmung durch das System 1 und dem Druck des Beatmungsgerätes 900 zusammensetzt.

Neben der Simulation einer aktiven Atmung, also der zumindest teilweise eigenständigen Atmung, eines Lebewesens ist in manchen Ausführungsformen des Systems vorgesehen, dass auch eine zumindest teilweise passive Atmung, also eine Vorgabe der Atmung beispielsweise durch das angeschlossene Beatmungsgerät 900, simulierbar ist. In manchen Ausführungsformen wird dazu die Vakuumpumpe 1004 entsprechend geregelt und/oder es ist ein Bypass vorgesehen, über welchen das von dem Beatmungsgerät 900 geförderte Gas an der Vakuumpumpe 1004 vorbei zum Auslass geleitet wird.

Über den Anschluss 105 des Gasmoduls 100 können verschiedene Geräte und/oder Mittel mit gasleitend mit dem System 1 verbunden werden. Beispielhaft ist in Figur 2 ein Beatmungsgerät 900 über eine Verbindung 800 mit dem System 1 verbunden. Das Beatmungsgerät 900 entspricht beispielhaft einem Beatmungsgerät nach dem Stand der Technik.

Das System 1 umfasst weiter ein Steuermodul 200 zur mathematischen Simulation der Atmung bzw. des Lebewesens und Steuerung des Gasmoduls 100. Das Steuermodul 200 umfasst eine Steuereinheit 201, welche auf Basis von Steuersignalen, welche durch die Simulationseinheit 202 generiert werden, zur Steuerung des Gasmoduls 100, insbesondere der Gasquellen 1001, 1002, 1003 und des Ventils 103 sowie der Vakuumpumpe 1004, ausgebildet und eingerichtet ist.

Die Sensoreinheit 203 ist dazu eingerichtet und ausgebildet, die von den Sensoren 1005, 1006, 1007, 1008 erfassten Messwerte aufzunehmen und ggf. weiterzuverarbeiten und/oder aufzubereiten. Die Auswerteeinheit 204 ist beispielhaft dazu eingerichtet und ausgebildet, die von der Sensoreinheit 203 aufgenommenen und ggf. weiterverarbeiteten Messwerte auszuwerten und optional zu analysieren. Es ist in manchen Ausführungsformen vorgesehen, dass die Simulationseinheit 202 die von der Sensoreinheit 203 aufgenommenen und/oder von der Auswerteeinheit 204 ausgewerteten Messwerte der mathematischen Simulation der Atmung zugrunde legt und gegebenenfalls die Steuersignale für die Steuereinheit 201 anpasst. Beispielsweise kann durch die Auswerteeinheit 204 und/oder die Simulationseinheit 202 festgestellt werden, dass die physikalische Simulation der Atmung nicht mit der mathematischen Simulation übereinstimmt. In dem Fall kann vorgesehen sein, dass die Simulationseinheit 202 und/oder die Steuereinheit 201 die Steuerung des Gasmoduls 100 entsprechend anpasst.

Weiter umfasst das System 1 eine Eingabeeinheit 205. Über die Eingabeeinheit 205 können Vorgaben, Einstellungen, Werte, Daten und/oder Informationen zur Simulation der Atmung eingegeben werden. Unter anderem können über die Eingabeeinheit 205 auch die Analysen, Auswertungen und/oder Messwerte der Sensoreinheit 203 bzw. der Auswerteeinheit 204 an die Simulationseinheit 202 weitergeleitet werden. Beispielhaft ist mit der Eingabeeinheit 205 ein Eingabemodul 300 verbunden, über welches Eingaben zur Simulation gemacht werden können und Daten, Werte und/oder Informationen zu der Simulation der Atmung ausgegeben und/oder angezeigt werden können. Beispielsweise ist die Eingabeeinheit 205 dazu als bidirektionale Schnittstelle ausgebildet, welche Daten empfangen und senden kann. Beispielsweise kann das Eingabemodul 300 über eine Kabel- und/oder Wireless-Verbindung mit der Eingabeeinheit 205 verbunden werden.

Beispielhaft umfasst das Steuermodul 200 eine Speichereinheit 206. In der Speichereinheit 206 können Messwerte, Analysen und/oder Auswertungen zumindest zwischengespeichert werden. In manchen Ausführungsformen des Systems 1 ist vorgesehen, dass sowohl die mathematische Simulation als auch die durch die Sensorik 104 erfassten Messwerte in der Speichereinheit 206 abgespeichert werden, beispielsweise für einen späteren Vergleich zwischen mathematischer und physikalischer Simulation. In manchen Ausführungsformen sind in der Speichereinheit 206 eine Vielzahl an Simulationsvorlagen und/oder Simulationsabläufen hinterlegt, auf welche über das Eingabemodul 300 zugegriffen werden kann.

Das Eingabemodul 300 ist insbesondere dazu eingerichtet, Vorgaben und/oder Einstellungen bezüglich der Simulation der Atmung in das System 1 einzugeben. Die Simulationseinheit 202 ist beispielsweise dazu eingerichtet und ausgebildet, anhand der Vorgaben und/oder Einstellungen die Atmung des Lebewesens mathematisch zu simulieren. Die Vorgaben und/oder Einstellungen umfassen beispielsweise aber nicht ausschließend Druck, Fluss, Lungenvolumen, Gaszusammensetzung, Atemfrequenz, Atemzugsvolumen, Art des Lebewesens, Alter, Gewicht, Krankheiten (insbesondere respiratorische Krankheiten), Gasaustausch, Atemprobleme. In manchen Ausführungsformen ist vorgesehen, dass das Eingabemodul 300 über eine Eingabemaske verfügt, über welche Einstellungen bezüglich der Simulation der Atmung eingegeben werden, welche an das Steuermodul 200 übermittelt werden.

Das in Figur 2 beispielhaft dargestellte System 1 ist auch dazu eingerichtet, zur physikalischen Simulation der Exspiration eine Gasmischung bereitzustellen, welche die Gaszusammensetzung der ausgeatmeten Luft eines Lebewesens simuliert. Dazu wird aus den Gasquellen 1001, 1002, 1003 über eine entsprechende Steuerung der Ventile ein Gasgemisch erzeugt. Beispielsweise kann so der Austausch von Sauerstoff und CO2 in der Lunge eines Lebewesens simuliert werden. In manchen Ausführungsformen ist vorgesehen, dass das Gas, welches während der simulierten Inspiration in das System 1 geleitet wird, aus dem System 1 über den Auslass 1014 ausgeleitet wird und für die Simulation der Exspiration ein frisches Gas bzw. Gasgemisch erzeugt wird. In manchen Ausführungsformen kann es auch vorgesehen sein, dass das Gas der Inspiration zur Exspirationseinheit 101 geleitet wird und dort aus den Gasquellen 1001, 1002, 1003 Gas zugemischt wird, sodass die Zusammensetzung einem ausgeatmeten Gas entspricht.

Zur Simulation des Gasaustausches in der Lunge kann vorgesehen sein, dass die Sensorik 104 auch einen Sensor zur Bestimmung der Gaszusammensetzung, insbesondere der Sauerstoffkonzentration und/oder CO2-Konzentration umfasst. Über die Analyse der Gaszusammensetzung während der simulierten Inspiration wird durch die Simulationseinheit 202, berechnet, wie die Gaszusammensetzung des Gases der simulierten Exspiration sein sollte. Auch ist es in manchen Ausführungsformen vorgesehen, dass Vorgaben bezüglich des simulierten Gasaustausches gemacht werden können. Beispielsweise kann einstellbar sein, dass eine geringe

Sauerstoffaufnahme in der Lunge simuliert werden soll. Entsprechend wird zur simulierten Exspiration ein Gasgemisch erzeugt, welches eine höhere Sauerstoffkonzentration aufweist als bei einem normalen Gasaustausch in der Lunge.

Neben den in Figur 2 dargestellten Gasquellen zur Bereitstellung von CO2, Sauerstoff und Stickstoff kann vorgesehen sein, dass das Exspirationsmodul 101 weitere Gasquellen umfasst, wie zum Beispiel eine oder mehrere Quellen für Anästhesiegas zur Simulation einer Aufnahme von Anästhesiegas in der Lunge. Ebenso können weitere Gasquellen vorgesehen sein, jeweils entsprechend der Gase, welche bei der Inspiration über den Anschluss 105 in das System 1 bzw. das Gasmodul 100 geleitet werden und deren Aufnahme über die Lunge und/oder die Luftwege simuliert werden sollen. Über das Steuermodul 200 und/oder das Eingabemodul 300 ist dabei eine Einstellung möglich, wie viel des Gases aufgenommen wird, wobei die entsprechende Gaszusammensetzung für die simulierte Exspiration berechnet wird und das Exspirationsmodul 101 entsprechend angesteuert wird.

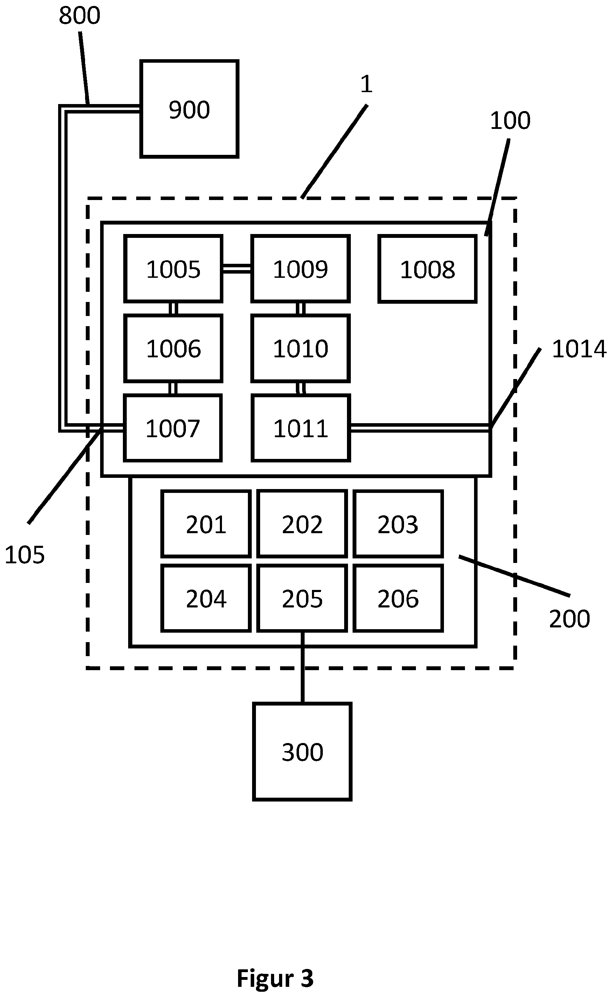

Eine weitere beispielhafte Ausführungsform des Systems 1 ist schematisch in Figur 3 dargestellt. Für den zweiten Simulationsteil, hier die physikalische Simulation der Atmung, sind in dem Gasmodul 100 zwei Gebläse 1010, 1011 angeordnet. Alternativ oder ergänzend zu den zwei Gebläsen 1010, 1011 kann auch zumindest eine bidirektionale Pumpe eingesetzt werden.

Die Förderrichtung der Gebläse 1010, 1011 ist dabei entgegengesetzt, sodass sowohl die Inspiration als auch die Exspiration physikalisch simuliert werden kann. Beispielsweise fungiert das Gebläse 1010 als Exspirationseinheit 101. Mit Förderrichtung ist dabei insbesondere die Druckrichtung gemeint. Beispielsweise baut das Gebläse 1010 einen Druck in Richtung Anschluss 105 bzw. Beatmungsgerät 900 auf. Das Gebläse 1010 saugt dazu beispielsweise Gas über den Auslass 1014 an und fördert das Gas durch das Gasmodul 100 zum Anschluss 105. Die Inspirationseinheit 102 wird beispielsweise über das Gebläse 1011 dargestellt, welches dazu eingerichtet ist entgegen der Förderrichtung des Gebläses 1010 Gas zu fördern. Durch die entgegengesetzte Förderrichtung, also ein Ansaugen von Gas am Anschluss 105, kann beispielsweise das einsaugen von Luft in die Luftröhre/Lunge eines Lebewesens simuliert werden. Die Stärke der Inspiration und der Exspiration, beispielsweise in Form von Druck und/oder Fluss, lässt sich unteranderem durch die Geschwindigkeit der Gebläse 1010, 1011 einstellen. Das Atemzugsvolumen ist entsprechend über die Dauer der Förderung kontrollierbar.

Zusätzlich zu den Gebläsen 1010, 1011 kann zur präziseren und/oder umfassenderen Simulation der Atmung ein pneumatischer Widerstand 1009 in dem Gasmodul 100 angeordnet sein. Beispielsweise können über den pneumatischen Widerstand 1009 spezifische Verhältnisse von Druck und Fluss erreicht werden. In manchen Ausführungsformen dient der pneumatische Widerstand 1009 primär einer besseren Steuerbarkeit des Flusses. In manchen Ausführungsformen ist der pneumatische Widerstand 1009 dazu steuerbar. Je nach zu erreichendem Druck/Fluss-Verhältnis kann der pneumatische Widerstand 1009 angepasst werden. So ist es zum Beispiel möglich, eine Vielzahl von verschiedenen Atmungen physikalisch zu simulieren. In manchen Ausführungsformen kann vorgesehen sein, dass auf einen pneumatischen Widerstand 1009 verzichtet wird oder als feststehender Widerstand eingesetzt ist, wobei ein variabler Widerstand durch die Gebläse erzeugt wird.

Über die Steuereinheit 201 lassen sich insbesondere die Gebläse 1010 und 1011 ansteuern. Beispielsweise wird zur Inspiration ausschließlich das Gebläse 1011 aktiviert, welches so angeordnet ist, dass es zur Simulation einer aktiven Inspiration, also die Simulation einer eigenständigen Inspiration des Lebewesens, Gas durch den Anschluss 105 ansaugt. Ist vorgesehen, dass eine vollständig passive Inspiration des Lebewesens simuliert werden soll, also kein eigener Antrieb der Inspiration, so kann das Gebläse 1011 während der Inspirationsphase stillstehen bzw. deaktiviert sein und ein Bypass (nicht dargestellt) geöffnet werden, welcher das vom Beatmungsgerät 900 (oder einer anderen Beatmungsquelle) geförderte Gas/Gasgemisch, z.B. Atemgas, an den Gebläsen 1010, 1011 vorbei direkt zum Auslass 1014 leitet. Während der simulierten Exspiration bleibt das Gebläse 1010 deaktiviert.

Auch ohne Bypass kann die passive Inspiration, also eine Beatmung durch das Beatmungsgerät 900, physikalisch simuliert werden. Beispielsweise arbeitet einer der Gebläse 1010, 1011 mit einem Druck gegen die Beatmung, etwa um eine Compliance nachzustellen. Bei der passiven Beatmung kann dann vorgesehen sein, dass das durch das Beatmungsgerät 900 geförderte Gas durch das andere, beispielsweise stillstehende Gebläse strömen kann.

Für eine simulierte Exspiration wird über die Steuereinheit 201 beispielhaft das Gebläse 1010 so angesteuert, dass ein beispielsweise voreingestelltes Exspirationsprofil, zumindest Fluss und Druck betreffend, simuliert wird. Das Gebläse 1011, welches für die Simulation der Inspiration verwendet wird, bleibt während der Exspirationssimulation deaktiviert. Zur Simulation der Exspiration ist das Gebläse 1010 dazu eingerichtet und ausgebildet,

Gas beispielsweise durch den Auslass 1014 anzusaugen und zum Anschluss 105 zu fördern. Über eine Variation der Förderleistung des Gebläses 1010, optional in Kombination mit einem pneumatischen Widerstand 1009, kann dann ein Exspirationsprofil simuliert werden. Beispielsweise wird zu Beginn der simulierten Exspiration ein hoher Fluss erzeugt, welcher im Laufe der Exspirationsphase abnimmt.

Die Gebläse 1010, 1011 sind beispielsweise so eingerichtet und angeordnet, dass Gas ungehindert entgegen der Förderrichtung durch die Gebläse strömen kann ohne diese, beispielsweise durch erzwungene Rotation der Förderräder entgegen der angedachten Richtung, beschädigt werden. In manchen Ausführungsformen ist vorgesehen, dass Bypass-Leitungen in dem Gasmodul 100 so angeordnet sind, dass Gas an den Gebläsen vorbeiströmen kann, während das jeweilige Gebläse deaktiviert ist.

Während die Steuereinheit 201 dazu eingerichtet ist, die Gebläse 1010, 1011 und ggf. den pneumatischen Widerstand 1009 so anzusteuern, dass die Vorgaben der mathematisch simulierten Atmung erreicht werden, wird die dadurch umgesetzte physikalisch simulierte Atmung über die Sensorik 104, beispielhaft über den Drucksensor 1005, den Flusssensor 1006 und den Temperatursensor 1007, überprüft. Die Auswerteeinheit 204 ist beispielsweise dazu eingerichtet und ausgebildet, die Messerwerte der Sensoren 1005, 1006, 1007 auszuwerten und daraufhin zu analysieren, ob die Beatmung entsprechend der Vorgaben simuliert wird. Insbesondere über den Flusssensor 1006 wird überprüft, ob die Beatmung entsprechend der Vorgaben (physikalisch) simuliert wird. Als Vorgabe dient beispielsweise der Fluss der mathematischen Simulation.

Beispielsweise ist die Simulationseinheit 202, gegebenenfalls in Kombination mit der Auswerteeinheit 204, dazu eingerichtet und ausgebildet, die physikalische Simulation der Atmung mit der mathematischen Simulation zu vergleichen und auf Abweichungen zu überprüfen. In manchen Ausführungsformen ist die Simulationseinheit 202 und/oder die Steuereinheit 201 dazu eingerichtet, etwaige Korrekturen der physikalischen Simulation automatisch durchzuführen. Die Simulationseinheit 202 ist beispielsweise auch dazu eingerichtet und ausgebildet, Gasparameter, beispielsweise Druck und/oder Fluss und/oder Temperatur und/oder Gaszusammensetzung, mit in die mathematische Simulation einzubeziehen, welche von einer externen Quelle, beispielsweise dem Beatmungsgerät 900, in das System 1 eingebracht werden. Beispielsweise wird bei der mathematischen Simulation durch die Simulationseinheit 202 der vom Beatmungsgerät 900 erzeugte Druck und/oder Fluss mit einbezogen.

Zur Erzeugung eines Atemgases, welches beispielsweise den Verbrauch und Erzeugung von Atemgasbestandteilen physikalisch simuliert kann insbesondere eine zumindest teilweise Kombination mit der in Figur 2 beschriebenen Ausführungsform vorgesehen sein. Beispielsweise wird die physikalische Simulation der Inspiration und Exspiration bzw. generell der Atembewegung durch die Gebläse 1010, 1011 realisiert, während das Gasgemisch, beispielsweise als Atemgas, durch zumindest eine Gasquelle bereitgestellt bzw. eingestellt wird. Ergänzend kann vorgesehen sein, dass ein Mischbereich, etwa eine Mischkammer, vorgesehen ist, um das vom Beatmungsgerät 900 gelieferte Atemgas mit dem von der mindestens einen Gasquelle bereitgestelltem Gas effektiv zu mischen. Entsprechend kann dabei auch vorgesehen sein, dass das im Bereich der Mischkammer, etwa am Ein-/Ausgang und/oder in der Mischkammer selbst, entsprechende Gassensoren angeordnet sind, um die Gaszusammensetzung zu überwachen. Die Einspeisung des zusätzlichen Gases kann beispielsweise zwischen den beiden Gebläsen 1010, 1011 und/oder vor und/oder hinter den beiden Gebläsen 1010, 1011 erfolgen. Insbesondere kann vorgesehen sein, dass die Einspeisung zwischen den Gebläsen und dem Anschluss des Beatmungsgerätes 900 erfolgt.

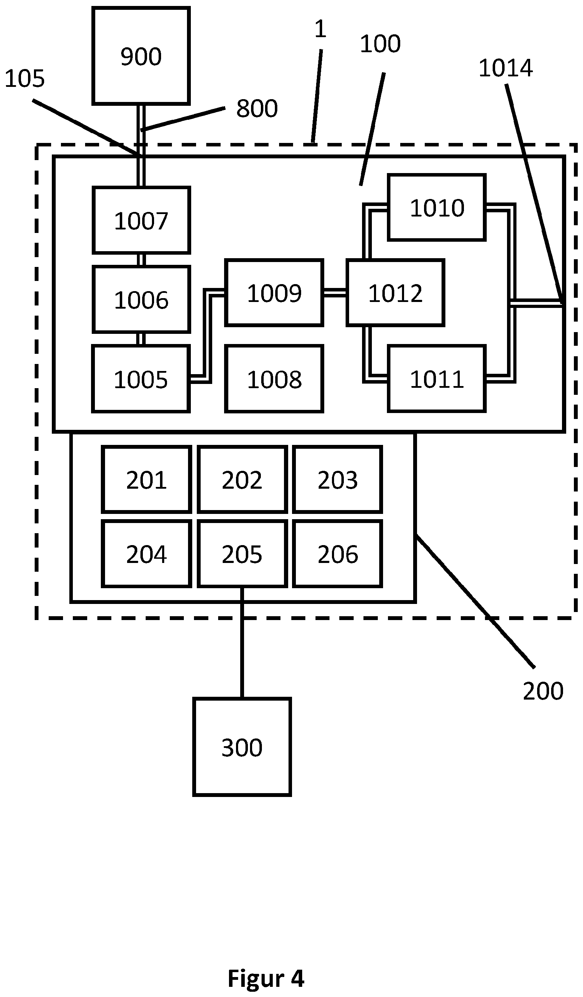

Figur 4 zeigt eine beispielhafte Ausführungsform des Systems 1 in Verbindung mit einem Beatmungsgerät 900, welches über eine Verbindung 800 mit dem Anschluss 105 an das System 1 angeschlossen ist. Ähnlich zu der in Figur 3 beschriebenen Ausführungsform wird die physikalische Simulation der Atmung auch hier über zwei Gebläse 1010, 1011 realisiert. In der in Figur 4 gezeigten Ausführungsform sind die Gebläse 1010, 1011 dazu parallel zueinander angeordnet. Ein Ventil 1012 ist so in dem Gasmodul 100 angeordnet, dass eine Schaltung zwischen den Gebläsen 1010, 1011 möglich ist.

Beispielsweise wird die Exspiration durch das Gebläse 1010 simuliert. Das System 1 ist dabei so eingerichtet und ausgebildet, dass die Steuereinheit 201 das Ventil 1012 so schaltet, dass ein Gasfluss vom Auslass 1014 zum Anschluss 105 über das Gebläse 1010 möglich ist. Zur Simulation der Exspiration fördert das Gebläse 1010 Gas vom Auslass 1014 zum Anschluss 105.

Zur Simulation der Inspiration, wird das Ventil 1012 so geschaltet, dass ein Gasfluss von dem Anschluss 105 über das Gebläse 1011 zum Auslass 1014 möglich ist. Bei der Simulation einer zumindest teilweise aktiven Inspiration, sodass zumindest die Atemanstrengung des Lebewesens simuliert wird, wird entsprechend das Gebläse 1011 aktiviert. Das Gebläse 1011 ist so eingerichtet und angeordnet, dass Gas vom Anschluss 105 angesaugt und zum Auslass 1014 gefördert wird. Über die Sensorik 104, insbesondere durch den Flusssensor 1006, kann dabei die Menge an Gas bestimmt werden, während der simulierten Inspiration in das System 1 strömt. Entsprechend eines voreingestellten, simulierten Lungenvolumen kann so beispielsweise gesteuert werden, wie lange und/oder mit welchem Fluss die Inspiration simuliert wird.

Bei der Simulation einer zumindest teilweise passiven Inspiration, bei der im Wesentlichen das Beatmungsgerät 900 die Inspiration bestimmt, wird das Gebläse 1011 beispielsweise deaktiviert. In manchen Ausführungsformen ist vorgesehen, dass das Gebläse 1011 zumindest einen leichten Fluss bzw. Druck erzeugt, um eine Atemanstrengung zu simulieren. Schaltet das Beatmungsgerät 900 auf die Beatmung, so kann vorgesehen sein, dass über eine Bypass-Leitung im Gasmodul 100 das vom Beatmungsgerät geförderte Gas an dem Gebläse 1011 vorbei zum Auslass 1014 geleitet wird.

Wird ein vorgegebenes simuliertes Lungenvolumen erreicht, so kann vorgesehen sein, dass das Ventil 1012 so geschlossen wird, dass kein weiteres Gas durch den Anschluss 105 in das Gasmodul 100 gefördert werden kann. In manchen Ausführungsformen kann vorgesehen sein, dass bei erreichtem simulierten Lungenvolumen auch das Gebläse 1010 aktiviert wird, um einen Gegendruck zu simulieren, welcher eine vollständige Ausdehnung bzw. Füllung der Lunge simuliert. Es kann auch vorgesehen sein, dass das Ventil 1012 dann umgeschaltet wird, wenn die mathematische Simulation vorgibt, dass der vom Lebewesen erzeugte Druck Null erreicht, also das Lebewesen die Einatmung oder die Ausatmung abgeschlossen hat und zur nächsten Atemphase übergeht.

Alternativ oder ergänzend kann auch der pneumatische Widerstand 1009 variabel ausgelegt sein, sodass der Flusswiderstand erhöht wird, was eine zunehmend gefüllte Lunge simuliert. In manchen Ausführungsformen kann auch ein fester pneumatischer Widerstand 1009 vorgesehen sein, welche zumindest der Stabilisierung der Flussregulierung dient.

Zur Steuerung des Gasmoduls 100 und zur mathematischen Simulation der Atmung ist in dem System 1 das Steuermodul 200 vorgesehen. Die Simulationseinheit 202 erstellt eine mathematische Simulation der Atmung, beispielsweise auf Basis von Vorgaben, welche beispielsweise durch das Eingabemodul 300, welche mit der Eingabeeinheit 205 des Steuermoduls 200 verbunden ist. Auch die durch die Sensorik 104, umfassend die Sensoren 1005, 1006, 1007, erfassten und durch die Sensoreinheit 203 und/oder Auswerteeinheit 204 aufgenommenen und gegebenenfalls ausgewerteten Messwerte können in manchen Ausführungsformen durch die Simulationseinheit 202 in die mathematische Simulation der Atmung einbezogen werden. Es ist auch vorgesehen, dass in der Speichereinheit 206 Simulationsvorlagen und/oder Simulationsabläufe hinterlegt sind, welche über das Eingabemodul abgerufen werden können und optional anpassbar sind. Die Simulationsvorlagen und/oder Simulationsabläufe umfassen beispielsweise Informationen/Daten zum zu simulierenden Lebewesen. Auch kann vorgesehen sein, dass in den Simulationsabläufen erfasst ist, ob und/oder welche Atemereignisse, beispielsweise Apnoen und/oder Atemwegsblockaden und/oder Änderungen in Druck/Fluss/Frequenz/Volumen der Atmung, simuliert werden sollen.

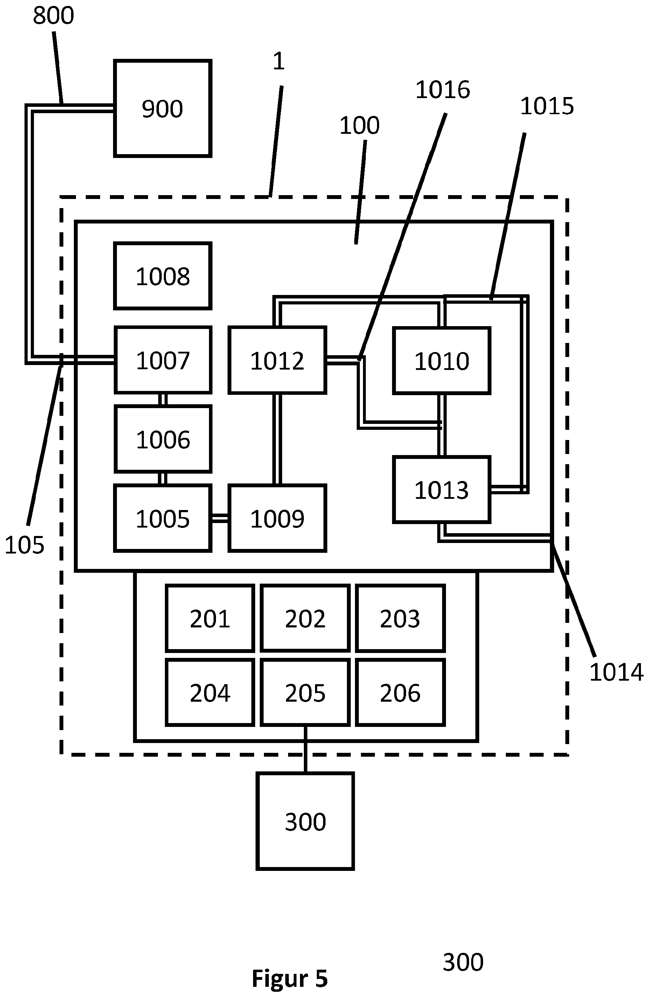

Figur 5 zeigt eine schematische Darstellung einer weiteren beispielhaften Ausführungsform des Systems 1 zur Simulation der Atmung eines Lebewesens. Die physikalische Simulation der Atmung im zweiten Simulationsteil, also sowohl die simulierte Inspiration als auch die simulierte Exspiration, wird durch das Gebläse 1010 in Kombination mit den Ventilen 1012 und 1013 simuliert. Das Gebläse 1010 stellt zusammen mit den Ventilen 1012 und 1013 eine kombinierte Exspirationseinheit 101 und Inspirationseinheit 102 (vgl. Figur 1) dar.

Je nach Förderrichtung bzw. Druckrichtung des Gebläses 1010 wird über die Ventile 1012, 1013 entschieden, ob eine Inspiration oder eine Exspiration simuliert wird. Beispielsweise werden die Ventile 1012 und 1013 dann umgeschaltet, wenn der zu simulierende Druck Null erreicht. Der zu simulierende Druck ist dabei jener Druck, welcher in der mathematischen Simulation dem durch das zu simulierende Lebewesen erzeugte Druck entspricht. Dies entspricht weitestgehend der natürlichen Atmung eines Menschen/Säugetiers, welches bei der Ausatmung einen positiven Druck in der Lunge erzeugt, durch welchen das Gas aus der Lunge gedrückt wird und bei der Einatmung über einen Unterdruck Gas in die Lunge saugt. Hierbei ist zu beachten, dass so der durch die Lunge bzw. das Lebewesen erzeugte Druck beschrieben wird. Ist beispielsweise ein Beatmungsgerät 900 mit dem Gasmodul verbunden, welches zusätzlich einen Druck erzeugt, so wird der durch die Simulation erzeugte Druck zusätzlich mit dem Druck des Beatmungsgerätes 900 beaufschlagt. So kann es auch vorkommen, dass innerhalb des Gasmoduls konstant ein positiver Druck gemessen wird. Durch die Simulation der Atmung schwankt, je nach Kompensation durch das Beatmungsgerät 900, der Druck um den vom Beatmungsgerät 900 erzeugten Druck.

Die Ventil 1012, 1013 sind dazu eingerichtet, das Gas durch die Bypass-Leitungen 1015, 1016 zu leiten. Das Gebläse 1010 ist beispielhaft so eingerichtet und ausgebildet, dass bei einer ersten Ventilstellung der Ventile 1015, 1016 Gas vom Auslass 1014 und durch das Ventil 1013 angesaugt wird und dann durch das Ventil 1012 zum Anschluss 105 gefördert wird. Das Gas wird dabei nicht durch die Bypass-Leitungen 1015, 1016 geleitet. Durch diese Förderrichtung und Gasleitung wird die Exspiration des Lebewesens simuliert.

In einer zweiten Ventilstellung sind die Ventile 1012, 1013 so gestellt, dass das Gas durch die Bypass-Leitungen 1015, 1016 geleitet wird. Dadurch saugt das Gebläse 1010 Gas vom Anschluss 105 über das Ventil 1012 durch die Bypass-Leitung 1016 an und fördert das Gas über die Bypass-Leitung 1015 durch das Ventil 1013 zum Auslass 1014. In dieser zweiten Ventilstellung wird entsprechend eine zumindest teilweise aktive Inspiration des Lebewesens simuliert. Durch das Umstellen von der ersten Ventilstellung auf die zweite Ventilstellung kann eine Umkehr der Förderrichtung des Gases durch das Gasmodul 100 erreicht werden.

In manchen Ausführungsformen ist eine dritte Ventilstellung vorgesehen, bei der die Ventile 1012, 1013 so geschaltet sind, dass das Gas durch Ventil 1012 durch die Bypass-Leitung 1015 an dem Gebläse 1010 vorbei und durch das Ventil 1013 zum Auslass 1014 geleitet wird. Diese dritte Ventilstellung wird beispielsweise eingestellt, wenn eine zumindest teilweise passive Inspiration eines Lebewesens simuliert werden soll. In manchen Ausführungsformen wird in dieser dritten Ventilstellung das Ventil 1012 so geschaltet, dass das Gas in die Bypass-Leitung 1016 geleitet wird und durch das Ventil 1013 direkt zum Auslass 1014 geleitet wird. Diese Schaltung gibt die Möglichkeit, dass über das Gebläse 1010 zumindest geringfügig Gas von dem Anschluss 105 angesaugt werden kann, um beispielsweise die Anstrengung des Lebewesens zum Einatmen zu simulieren. Beispielweise kann so simuliert werden, dass das Lebewesen zwar eine Atemanstrengung zeigt, diese aber nicht ausreichend ist, um komplett selber einzuatmen und eine externe Beatmung, beispielsweise über das Beatmungsgerät 900, nötig ist.

Über den pneumatischen Widerstand 1009, optional variabel, lässt sich ein spezifisches Druck-zu-Fluss-Verhältnis realisieren. Ist der pneumatische Widerstand 1009 dazu variabel ausgeführt, kann so eine Vielzahl von Druck-zu-Fluss-Verhältnissen simuliert werden, beispielsweise um verschiedene Durchmesser der Luftröhre und/oder verschiedene Atemmuster bzw. Atemprobleme zu simulieren. In manchen Ausführungsformen dient der pneumatische Widerstand auch oder hauptsächlich der Stabilisierung der Flussregelung.

Über das Steuermodul 200, insbesondere die Steuereinheit 201, wird das Gasmodul 100 gesteuert. Die Steuersignale zur Steuerung werden zum Beispiel über die Simulationseinheit 202 aus der mathematischen Simulation der Atmung abgleitet. Über das Eingabemodul 300 werden beispielsweise die Vorgaben zur Simulation der Atmung eingegeben. Die Vorgaben können zum Beispiel Lungenvolumen, Fluss, Druck, Atemzugsvolumen, Atemfrequenz, den Simulationsablauf, Größe, Gewicht, Erkrankungen, etc. des Lebewesens, dessen Atmung simuliert werden soll, betreffen. In manchen Ausführungsformen können zumindest Eingaben hinsichtlich Druck und Fluss der zu simulierenden Atmung gemacht werden. In manchen Ausführungsformen ist das System 1 dazu ausgelegt neben der Atmung von Menschen auch die Atmung von anderen Lebewesen, insbesondere Säugetieren, zu simulieren. Dazu umfassten die einstellbaren Vorlagen beispielsweise auch eine Auswahl des jeweiligen Lebewesens.

In der Speichereinheit 206 können Vorlagen, beispielsweise für zu simulierende Lebewesen und/oder Simulationsabläufe, hinterlegt sein. Diese Vorlagen können beispielsweise angepasst werden. Beispielsweise können Simulationsabläufe hinterlegt sein, welche eine Vielzahl von Atemwegsproblemen enthalten. Beispielsweise kann so die Funktion des angeschlossenen Beatmungsgerätes 900 getestet werden. In manchen Ausführungsformen kann vorgesehen sein, dass die Simulation der Atmung auch während der Simulation über das Eingabemodul 300 angepasst werden kann.

Entsprechend der Vorgaben erfolgt durch die Simulationseinheit 202 in einem ersten Simulationsteil eine mathematische Simulation der Atmung. Aus der mathematischen Simulation der Atmung werden entsprechende Steuersignale abgeleitet und an die Steuereinheit 201 übermittelt. Anhand der Steuersignale steuert die Steuereinheit 201 in einem zweiten Simulationsteil das Gasmodul 100, beispielsweise um eine physikalische Simulation der Atmung umzusetzen.

Im Gasmodul 100 ist weiter eine Sensorik 104 (vgl. Figur 4) angeordnet, umfassend zumindest zwei Drucksensoren 1005, 1008, einen Flusssensor 1006 und einen Temperatursensor 1007. Der Drucksensor 1008 dient dabei der Messung des Umgebungsdrucks. Über die Sensoren 1005, 1006, 1007 werden Gasparameter wie Fluss, Druck, Temperatur gemessen. Die hier gemessenen Werte stellen dabei die Gasparameter dar, wie sie in der Lunge bzw. der Luftröhre des Lebewesens vorherrschen, dessen Atmung simuliert wird. Die Messwerte der Sensoren 1005, 1006, 1007, 1008 werden von der Sensoreinheit 203 aufgenommen und gegebenenfalls weiterverarbeitet und/oder aufbereitet. Die Auswerteeinheit 204 des Steuermoduls 200 ist dazu eingerichtet und ausgebildet, die von der Sensoreinheit 203 aufgenommenen Messwerte auszuwerten und/oder zu analysieren. In manchen Ausführungsformen ist das Steuermodul 200 so eingerichtet und ausgebildet, dass die Steuerung der Simulation anhand der Analysen/Auswertungen der Auswerteeinheit 204 angepasst werden. In manchen Ausführungsformen werden die Analysen/Auswertungen über die Eingabeeinheit 205 an die Simulationseinheit 202 weitergeleitet, sodass dort eine mögliche Anpassung der Simulation erfolgt und entsprechend angepasste Steuersignale an die Steuereinheit 201 übermittelt werden.

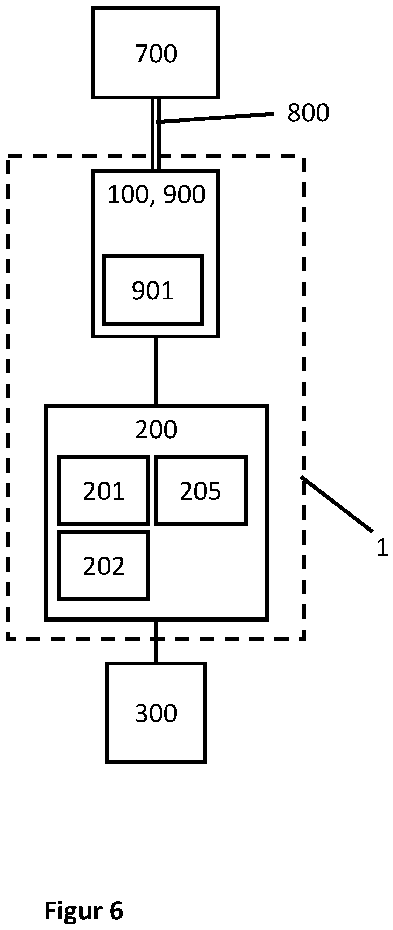

Figur 6 zeigt eine beispielhafte Ausführungsform des Systems 1, wobei als Gasmodul 100 ein Beatmungsgerät 900 eingesetzt ist und über die Verbindung 800 ein realer Patient 700 mit dem System 1 verbunden ist. Das Beatmungsgerät 900 kann dabei ein Beatmungsgerät nach dem Stand der Technik sein.

Das System 1 ist dazu eingerichtet, dass das Beatmungsgerät 900 anhand der mathematischen Simulation der Atmung des angeschlossenen Patienten 700 gesteuert wird. Über Sensoren werden Daten und Werte zur Atmung des Patienten 700 aufgenommen. In manchen Ausführungsformen ist dazu vorgesehen, dass das Beatmungsgerät 900 über entsprechende Sensoren und optional auch Mittel zur Weiterverarbeitung und Auswertung aufweist. Die Werte zur Atmung des Patienten 700 werden über die Eingabeeinheit 205 an die Simulationseinheit 202 weitergeleitet. Die Simulationseinheit 202 ist dazu eingerichtet und ausgebildet, anhand der Werte des Patienten 700, den Patienten 700 in einem ersten Simulationsteil mathematisch zu simulieren. Die mathematische Simulation umfasst beispielsweise auch die Simulation der Lunge bzw. Lungenwerte und/oder auch weiterer Vitalwerte des Patienten 700. In manchen Ausführungsformen ist vorgesehen, dass über die mathematische Simulation Werte des Patienten berechnet bzw. simuliert werden, welche beispielsweise über die Sensoren nicht zugänglich sind.

Beispielhaft ist für das in Figur 6 dargestellte System 1 vorgesehen, dass das Beatmungsgerät 900 zumindest teilweise anhand der mathematischen Simulation des Patienten 700 gesteuert wird. Dazu ist die Simulationseinheit 202 dazu eingerichtet, aus der mathematischen Simulation Steuersignale abzuleiten. Die Steuersignale werden an die Steuereinheit 201 weitergeleitet, welche dazu eingerichtet ist entweder direkt das Beatmungsgerät 900 zu steuern oder diese Steuersignale an eine Beatmungssteuerung 901 weiterzuleiten. In diesem zweiten Simulationsteil wird die Beatmung des Patienten 700 also anhand der mathematischen Simulation des ersten Simulationsteils gesteuert.

### Bezugszeichenliste

- 1: System
- 100: Gasmodul
- 101: Exspirationssimulator
- 102: Inspirationssimulator
- 103: Ventil
- 104: Sensorik
- 105: Anschluss
- 200: Steuermodul
- 201: Steuereinheit
- 202: Simulationseinheit
- 203: Auswerteeinheit
- 204: Sensoreinheit
- 205: Eingabeeinheit
- 206: Speichereinheit
- 300: Eingabemodul
- 700: Patient
- 800: Verbindung
- 900: Beatmungsgerät
- 1001: CO2 Quelle
- 1002: O2 Quelle
- 1003: N2 Quelle
- 1004: Vakuumpumpe
- 1005: Drucksensor
- 1006: Flusssensor
- 1007: Temperatursensor
- 1008: Umgebungsdrucksensor
- 1009: pneumatischer Widerstand
- 1010: Gebläse
- 1011: Gebläse
- 1012: 2-Wege Ventil
- 1013: 2-Wege Ventil
- 1014: Auslass
- 1015: Saug-Bypass
- 1016: Saug-Bypass
- 9001: Beatmungssteuerung

## Patentansprüche

1. System (1) zur Simulation der Atmung eines Lebewesens, umfassend zumindest ein Gasmodul (100) und ein Steuermodul (200), wobei das Steuermodul (200) dazu eingerichtet und ausgebildet ist, in einem ersten Simulationsteil die Atmung eines Lebewesens mathematisch zu simulieren und in einem zweiten Simulationsteil anhand der mathematischen Simulation aus dem ersten Simulationsteil das Gasmodul (100) zu steuern, wobei Atmungssituationen, beispielsweise Apnoen, Kollaps der Alveolen, Schnappatmung, erhöhte oder verringerte Atemfrequenz oder Situationen, die einem Schnarchen gleichen, im ersten Simulationsteil vorprogrammiert sind und vorgegeben werden kann, welche der Atmungssituationen simuliert werden soll oder sollen, **dadurch gekennzeichnet, dass** die mathematische Simulation einen Zufallsgenerator umfasst, der konfiguriert ist, um die zu simulierende Atmungssituation oder die zu simulierenden Atmungssituationen zufällig in die mathematische Simulation mit einzubeziehen.

2. System (1) nach Anspruch 1, wobei ferner eine Ausprägung der zu simulierenden Atmungssituation oder der zu simulierenden Atmungssituationen vorgegeben werden kann.

3. System (1) nach Anspruch 2, wobei der Zufallsgenerator konfiguriert ist, um die Ausprägung vorzugeben.

4. System (1) nach zumindest einem der vorhergehenden Ansprüche, wobei der Zufallsgenerator konfiguriert ist, um mindestens einen der folgenden Parameter zufällig um einen Mittelwert schwanken zu lassen: Druck, Fluss, Gaszusammensetzung, Atemzugsvolumen.

5. System (1) nach zumindest einem der vorhergehenden Ansprüche, wobei das Steuermodul (200) dazu eingerichtet und ausgebildet ist, das Gasmodul (100) so anzusteuern, dass in dem zweiten Simulationsteil die mathematische Simulation des ersten Simulationsteils in eine physikalische Simulation der Atmung eines Lebewesens umgesetzt wird.

6. System (1) nach zumindest einem der vorhergehenden Ansprüche, wobei das Gasmodul (100) zumindest eine Exspirationseinheit (101) und zumindest eine Inspirationseinheit (102) umfasst, wobei die Exspirationseinheit (101) dazu eingerichtet ist, eine Exspiration eines Lebewesens zu simulieren, und die Inspirationseinheit (102) dazu eingerichtet ist, eine Inspiration eines Lebewesens zu simulieren.

7. System (1) nach Anspruch 6, wobei die Exspirationseinheit (101) zumindest eine Gasquelle (1001, 1002, 1003) und/oder zumindest ein Gebläse umfasst.

8. System (1) nach Anspruch 6 oder 7, wobei die Exspirationseinheit (101) eine Mehrzahl von Gasquellen (1001, 1002, 1003) umfasst und dazu eingerichtet und ausgebildet ist, auf Basis der mathematischen Simulation eine Gasmischung bereitzustellen, die einer Gaszusammensetzung der ausgeatmeten Luft eines Lebewesens entspricht.

9. System (1) nach zumindest einem der vorhergehenden Ansprüche, wobei das Steuermodul (200) eine Simulationseinheit (202) umfasst, die dazu eingerichtet und ausgebildet ist, die Atmung mathematisch zu simulieren und einen Druck, der durch das simulierte Lebewesen in der Lunge erzeugt wird, zu berechnen und/oder zu simulieren.

10. System (1) nach zumindest einem der vorhergehenden Ansprüche, wobei das Gasmodul (100) dazu ausgebildet und eingerichtet ist, einen Druck, der durch das simulierte Lebewesen in der Lunge erzeugt wird, physikalisch zu simulieren.

11. System (1) nach zumindest einem der vorhergehenden Ansprüche, wobei das Gasmodul (100) über einen Anschluss (105) mit einem Beatmungsgerät (900) verbindbar ist.

12. System (1) nach zumindest einem der vorhergehenden Ansprüche, wobei ein mathematisch simulierter Atemfluss durch zumindest ein Gebläse physikalisch simuliert wird und die simulierte Gaszusammensetzung durch zumindest eine Gasquelle (1001, 1002, 1003) erreicht wird.

13. System (1) nach zumindest einem der vorhergehenden Ansprüche, wobei in dem Gasmodul (100) ein Gebläse (1010) angeordnet ist, wobei über eine Schaltung von im Gasmodul (100) angeordneten Ventilen (1012, 1013) und Bypass-Leitungen (1015, 1016) das Gebläse (1010) sowohl als Exspirationseinheit (101) als auch als Inspirationseinheit (102) dient.

14. System (1) nach zumindest einem der vorhergehenden Ansprüche, wobei das System (1) eine Sensorik (104) umfasst, die dazu eingerichtet und ausgebildet ist, Werte der Atmung zu erfassen, wobei das Steuermodul (200) dazu eingerichtet und ausgebildet ist, die über die Sensorik (104) aufgenommenen Werte in die mathematische Simulation mit einzubeziehen, wobei das Steuermodul (200) eine Auswerteeinheit (204) umfasst, die dazu eingerichtet und ausgebildet ist, die über die Sensorik (104) erfassten Werte auszuwerten und/oder zu analysieren.

15. System (1) nach Anspruch 14, wobei die Auswerteeinheit (204) dazu eingerichtet und ausgebildet ist, die über die Sensorik (104) erfassten Werte dahingehend zu analysieren, ob die mathematische Simulation durch das Gasmodul (100) korrekt umgesetzt wird.

16. System (1) nach zumindest einem der vorhergehenden Ansprüche, wobei das System (1) über eine Eingabeeinheit (205) verfügt, über die Daten, Werte und/oder Informationen eingegeben werden können, wobei die Daten, Werte und/oder Informationen zumindest teilweise als Vorgaben zur mathematischen Simulation dienen.

17. System (1) nach Anspruch 16 rückbezogen auf die Ansprüche 9 und 14, wobei die Eingabeeinheit (205) dazu eingerichtet und ausgebildet ist, Werte und/oder Daten und/oder Informationen aus der Auswerteeinheit (204) in die Simulationseinheit (202) einzugeben.

18. System (1) nach zumindest einem der vorhergehenden Ansprüche, wobei das Steuermodul (200) dazu eingerichtet und ausgebildet ist, ein Beatmungsgerät (900) anhand der mathematischen Simulation zumindest teilweise zu steuern, wobei das Beatmungsgerät (900) mit einer realen Person verbunden ist.

19. Verfahren zur Simulation der Atmung eines Lebewesens mittels eines Systems nach zumindest einem der vorhergehenden Ansprüche, wobei das Verfahren umfasst: Simulieren der Atmung des Lebewesens im ersten Simulationsteil durch eine mathematische Simulation, wobei vorgegeben wird, welche der im ersten Simulationsteil vorprogrammierten Atmungssituationen simuliert werden soll oder sollen, und der Zufallsgenerator die zu simulierende Atmungssituation oder die zu simulierenden Atmungssituationen zufällig in die mathematische Simulation mit einbezieht;
Steuern des Gasmoduls (100) im zweiten Simulationsteil auf Basis der mathematischen Simulation.

20. Verfahren nach Anspruch 19, wobei in einem Verfahrensschritt über Sensoren Messwerte zur Atmung aufgenommen werden und in die mathematische Simulation mit einbezogen werden, wobei die mathematische Simulation automatisch anhand der aufgenommenen Messwerte angepasst und/oder abgeändert wird.

## Claims

1. A system (1) for simulating the breathing of a living being, comprising at least one gas module (100) and a control module (200), wherein the control module (200) is configured and designed to mathematically simulate the breathing of a living being in a first simulation part and, based on the mathematical simulation from the first simulation part, to control the gas module (100) in a second simulation part, wherein breathing situations, for example apnea, collapse of the alveoli, gasping, increased or decreased breathing frequency, or situations that resemble snoring, are preprogrammed in the first simulation part and it can be specified which of the breathing situations is or are to be simulated, **characterized in that** the mathematical simulation comprises a random generator which is configured to randomly include the breathing situation to be simulated or the breathing situations to be simulated in the mathematical simulation.

2. The system (1) according to claim 1, wherein, furthermore, a severity of the breathing situation to be simulated or the breathing situations to be simulated can be specified.

3. The system (1) according to claim 2, wherein the random generator is configured to specify the severity.

4. The system (1) according to at least one of the preceding claims, wherein the random generator is configured to let at least one of the following parameters fluctuate randomly around an average: pressure, flow, gas composition, breath volume.

5. The system (1) according to at least one of the preceding claims, wherein the control module (200) is configured and designed to actuate the gas module (100) such that, in the second simulation part, the mathematical simulation of the first simulation part is converted into a physical simulation of the breathing of a living being.

6. The system (1) according to at least one of the preceding claims, wherein the gas module (100) comprises at least one expiration unit (101) and at least one inspiration unit (102), wherein the expiration unit (101) is configured to simulate an expiration of a living being, and the inspiration unit (102) is configured to simulate an inspiration of a living being.

7. The system (1) according to claim 6, wherein the expiration unit (101) comprises at least one gas source (1001, 1002, 1003) and/or at least one fan.

8. The system (1) according to claim 6 or 7, wherein the expiration unit (101) comprises a plurality of gas sources (1001, 1002, 1003) and is configured and designed to provide, on the basis of the mathematical simulation, a gas mixture which corresponds to a gas composition of the exhaled air of a living being.

9. The system (1) according to at least one of the preceding claims, wherein the control module (200) comprises a simulation unit (202) which is configured and designed to mathematically simulate the breathing and to calculate and/or simulate a pressure which is generated in the lungs of the simulated living being.

10. The system (1) according to at least one of the preceding claims, wherein the gas module (100) is designed and configured to physically simulate a pressure which is generated in the lungs of the simulated living being.

11. The system (1) according to at least one of the preceding claims, wherein the gas module (100) can be connected to a ventilator (900) via a connection (105).

12. The system (1) according to at least one of the preceding claims, wherein a mathematically simulated breath flow is physically simulated by at least one fan and the simulated gas composition is achieved by at least one gas source (1001, 1002, 1003).

13. The system (1) according to at least one of the preceding claims, wherein a fan (1010) is arranged in the gas module (100), wherein, by switching valves (1012, 1013) and bypass lines (1015, 1016) arranged in the gas module (100), the fan (1010) serves both as an expiration unit (101) and as an inspiration unit (102).

14. The system (1) according to at least one of the preceding claims, wherein the system (1) comprises a sensor system (104) which is configured and designed to detect values of the breathing, wherein the control module (200) is configured and designed to include values recorded by the sensor system (104) in the mathematical simulation, wherein the control module (200) comprises an evaluation unit (204) which is configured and designed to evaluate and/or analyze the values detected by the sensor system (104).

15. The system (1) according to claim 14, wherein the evaluation unit (204) is configured and designed to analyze the values detected by the sensor system (104) in order to determine whether the mathematical simulation is implemented correctly by the gas module (100).

16. The system (1) according to at least one of the preceding claims, wherein the system (1) has an input unit (205), via which data, values, and/or information can be input, wherein the data, values, and/or information serve at least in part as specifications for the mathematical simulation.

17. The system (1) according to claim 16 when dependent on claim 9 and 14, wherein the input unit (205) is configured and designed to input values and/or data and/or information from the evaluation unit (204) into the simulation unit (202).

18. The system (1) according to at least one of the preceding claims, wherein the control module (200) is configured and designed to at least partially control a ventilator (900) based on the mathematical simulation, wherein the ventilator (900) is connected to a real person.

19. A method for simulating the breathing of a living being by means of a system according to at least one of the preceding claims, wherein the method comprises: simulating the breathing of a living being in the first simulation part by means of a mathematical simulation, wherein it is specified which of the breathing situations preprogrammed in the first simulation part is or are to be simulated, and the random generator randomly includes the breathing situation to be simulated or the breathing situations to be simulated in the mathematical simulation; controlling the gas module (100) in the second simulation part on the basis of the mathematical simulation.

20. The method according to claim 19, wherein, in one method step, measured values with regard to the breathing are recorded by sensors and included in the mathematical simulation, wherein the mathematical simulation is adapted and/or modified automatically based on the recorded measured values.

## Revendications

1. Système (1) de simulation de la respiration d'un organisme vivant, comportant au moins un module de gaz (100) et un module de commande (200), dans lequel le module de commande (200) est configuré et conçu pour simuler mathématiquement la respiration d'un organisme vivant dans une première partie de simulation et pour commander le module de gaz (100) dans une deuxième partie de simulation à l'aide de la simulation mathématique issue de la première partie de simulation, dans lequel des situations respiratoires, par exemple l'apnée, une défaillance des alvéoles, une respiration agonale, une fréquence respiratoire accrue ou réduite, ou des situations semblables à un ronflement, sont préprogrammées dans la première partie de simulation et il est possible de prédéfinir laquelle ou lesquelles des situations respiratoires doit ou doivent être simulée(s), **caractérisé en ce que** la simulation mathématique comporte un générateur aléatoire, lequel est configuré pour intégrer de façon aléatoire, dans la simulation mathématique, la situation respiratoire à simuler ou les situations respiratoires à simuler.

2. Système (1) selon la revendication 1, dans lequel une intensité de la situation respiratoire à simuler ou des situations respiratoires à simuler peut être prédéfinie.

3. Système (1) selon la revendication 2, dans lequel le générateur aléatoire est configuré pour prédéfinir l'intensité.

4. Système (1) selon l'une au moins des revendications précédentes, dans lequel le générateur aléatoire est configuré pour laisser fluctuer au moins l'un des paramètres suivants de façon aléatoire autour d'une valeur moyenne : pression, débit, composition de gaz, volume courant.

5. Système (1) selon l'une au moins des revendications précédentes, dans lequel le module de commande (200) est configuré et conçu pour actionner le module de gaz (100) de manière à ce que dans la deuxième partie de simulation, la simulation mathématique de la première partie de simulation soit convertie en une simulation physique de la respiration d'un organisme vivant.

6. Système (1) selon l'une au moins des revendications précédentes, dans lequel le module de gaz (100) comporte au moins une unité d'expiration (101) et au moins une unité d'inspiration (102), dans lequel l'unité d'expiration (101) est configurée pour simuler une expiration d'un organisme vivant et l'unité d'inspiration (102) est configurée pour simuler une inspiration d'un organisme vivant.

7. Système (1) selon la revendication 6, dans lequel l'unité d'expiration (101) comporte au moins une source de gaz (1001, 1002, 1003) et/ou au moins un ventilateur.

8. Système (1) selon la revendication 6 ou 7, dans lequel l'unité d'expiration (101) comporte une pluralité de sources de gaz (1001, 1002, 1003) et est configurée et conçue pour fournir, sur la base de la simulation mathématique, un mélange de gaz correspondant à une composition de gaz de l'air exhalé d'un organisme vivant.

9. Système (1) selon l'une au moins des revendications précédentes, dans lequel le module de commande (200) comporte une unité de simulation (202), laquelle est configurée et conçue pour simuler la respiration mathématiquement et pour calculer et/ou simuler une pression produite dans les poumons par l'organisme vivant simulé.

10. Système (1) selon l'une au moins des revendications précédentes, dans lequel le module de gaz (100) est configuré et conçu pour simuler physiquement une pression produite dans les poumons par l'organisme vivant simulé.

11. Système (1) selon l'une au moins des revendications précédentes, dans lequel le module de gaz (100) peut être relié à un appareil respiratoire (900) par le biais d'un raccord (105).

12. Système (1) selon l'une au moins des revendications précédentes, dans lequel un débit respiratoire simulé mathématiquement est simulé physiquement par au moins un ventilateur et la composition de gaz simulée est atteinte par au moins une source de gaz (1001, 1002, 1003).

13. Système (1) selon l'une au moins des revendications précédentes, dans lequel un ventilateur (1010) est disposé dans le module de gaz (100), dans lequel le ventilateur (1010) sert aussi bien comme unité d'expiration (101) que comme unité d'inspiration (102), par commutation de valves (1012, 1013) et des conduites de dérivation (1015, 1016) disposées dans le module de gaz (100).

14. Système (1) selon l'une au moins des revendications précédentes, dans lequel le système (1) comporte un capteur (104), lequel est configuré et conçu pour détecter des valeurs de la respiration, dans lequel le module de commande (200) est configuré et conçu pour intégrer des valeurs enregistrées par le biais du capteur (104) dans la simulation mathématique, dans lequel le module de commande (200) comporte une unité d'évaluation (204), laquelle est configurée et conçue pour évaluer et/ou analyser les valeurs détectées par le biais du capteur (104).

15. Système (1) selon la revendication 14, dans lequel l'unité d'évaluation (204) est configurée et conçue pour analyser les valeurs détectées par le biais du capteur (104) de manière à déterminer si la simulation mathématique est appliquée correctement par le module de gaz (100).

16. Système (1) selon l'une des revendications précédentes, dans lequel le système (1) dispose d'une unité d'entrée (205), par le biais de laquelle des données, des valeurs et/ou des informations peuvent être entrées, dans lequel les données, valeurs et/ou informations servent au moins partiellement comme spécifications pour la simulation mathématique.

17. Système (1) selon la revendication 16 lorsqu'elle dépend des revendications 9 et 14, dans lequel l'unité d'entrée (205) est configurée et conçue pour entrer des valeurs et/ou des données et/ou des informations à partir de l'unité d'évaluation (204) dans l'unité de simulation (202).

18. Système (1) selon l'une au moins des revendications précédentes, dans lequel le module de commande (200) est configuré et conçu pour commander au moins partiellement un appareil respiratoire (900) à l'aide de la simulation mathématique, dans lequel l'appareil respiratoire (900) est relié à une personne réelle.

19. Procédé de simulation de la respiration d'un organisme vivant au moyen d'un système selon l'une au moins des revendications précédentes, dans lequel le procédé comporte :
la simulation de la respiration d'un organisme vivant par une simulation mathématique dans la première partie de simulation, dans lequel il est prédéfini, laquelle ou lesquelles des situations respiratoires préprogrammées dans la première partie de simulation doit ou doivent être simulée(s), et le générateur aléatoire intègre de façon aléatoire la situation respiratoire à simuler ou les situations respiratoires à simuler dans la simulation mathématique ;
la commande du module de gaz (100) dans la deuxième partie de simulation sur la base de la simulation mathématique.

20. Procédé selon la revendication 19, dans lequel, dans une étape de procédé, des valeurs de mesure destinées à la respiration sont enregistrées par le biais de capteurs et intégrées dans la simulation mathématique, dans lequel la simulation mathématique est adaptée et/ou modifiée automatiquement à l'aide des valeurs de mesure enregistrées.
